(19) Europäisches Patentamt
European Patent Office
Office européen des brevets

(11) Publication number : **0 629 697 A2**

# (12) EUROPEAN PATENT APPLICATION

(21) Application number : **94304432.1**

(22) Date of filing : **20.06.94**

(51) Int. Cl.$^5$ : **C12N 15/63,** A61K 31/00

(30) Priority : **21.06.93 US 81610**
**18.05.94 US 246990**

(43) Date of publication of application :
**21.12.94 Bulletin 94/51**

(84) Designated Contracting States :
**AT BE CH DE DK ES FR GB GR IE IT LI LU NL PT SE**

(71) Applicant : **ELI LILLY AND COMPANY**
**Lilly Corporate Center**
**Indianapolis Indiana 46285 (US)**

(72) Inventor : **Yang, Na Nora**
**2750 Wood Wind Way**
**Indianapolis, Indiana 46268 (US)**

(74) Representative : **Hudson, Christopher Mark et al**
**Lilly Industries Limited**
**European Patent Operations**
**Erl Wood Manor**
**Windlesham Surrey GU20 6PH (GB)**

## (54) Materials and methods for screening anti-osteoporosis agents.

(57) The present invention relates to methods for the identification of therapeutic agents for the treatment of osteoporosis and serum lipid lowering agents. The invention relates to isolating cloning, and using nucleic acids from the promoter regions of transforming growth factor β genes comprising novel regulatory elements designated "raloxifene responsive elements". The invention also encompasses eukaryotic cells containing such raloxifene responsive elements operably linked to reporter genes such that the raloxifene responsive elements modulate the transcription of the reporter genes. The invention provides methods for identifying anti-osteoporosis agents that induce transcription of genes operably linked to such raloxifene responsive elements without inducing deleterious or undesirable side effects associated with current anti-osteoporosis therapy regimens.

EP 0 629 697 A2

The invention relates to methods for identifying therapeutic agents for the treatment of osteoporosis. The invention relates to isolating, cloning, and using nucleic acids comprising the promoter regions of mammalian transforming growth factor β genes that are novel regulatory elements designated "raloxifene responsive elements". The invention also encompasses genetically engineered eukaryotic cells containing the recombinant expression constructs wherein the raloxifene responsive elements are operably linked to reporter genes. In such cells the raloxifene responsive elements are capable of modulating transcription of the reporter genes in response to treatment with certain compounds. The invention also relates to methods for identifying anti-osteoporosis agents that induce transcription of certain genes via raloxifene responsive elements and that specifically do not induce deleterious or undesirable side effects that have been associated with estrogen replacement therapy, such as increased risk with uterine and breast cancer. The nucleic acids, cells, and methods of the invention provide effective methods for screening putative sources of anti-osteoporosis agents, and identifying those that advantageously lack the undesirable side effects associated with current anti-osteoporosis agents. The invention also relates to a method for inducing bone formation, a method for treating osteoporosis, and a method for treating bone fractures which comprise administering a compound that, when bound to an estrogen receptor, potently induces transcription from a raloxifene responsive element.

In 1991, U.S. pharmaceutical companies spent an estimated $7.9 billion on research and development devoted to identifying new therapeutic agents (Pharmaceutical Manufacturer's Association). The magnitude of this amount is due, in part, to the fact that the hundreds, if not thousands, of chemical compounds must be tested in order to identify a single effective therapeutic agent that does not engender unacceptable levels of undesirable or deleterious side effects. There is an increasing demand for economical methods of testing large numbers of testing large number of chemical compounds to quickly identify those compounds that are likely to be effective in treating disease. At present, few such economical systems exist.

One disease that conspicuously lacks a rapid method for screening large numbers of potential therapeutic agents is bone loss. Bone loss occurs in a wide variety of patients, including those who have undergone hysterectomy, who are undergoing or have undergone long-term administration of corticosteroids, who suffer from Cushing's syndrome or have gonadal dysgenesis, as well as post-menopausal women.

The mechanism of bone loss is not well understood, but in practical effect, the disorder arises from an imbalance in the formation of new healthy bone and the resorption of old bone, skewed toward a net loss of bone tissue. This bone loss includes a decrease in both mineral content and protein matrix components of the bone, and leads to an increased fracture rate of the femoral bones and bones in the forearm and vertebrae predominantly. These fractures, in turn, lead to an increase in general morbidity, a marked loss of stature and mobility, and in many cases, an increase in mortality resulting from complications.

Unchecked, bone loss can lead to osteoporosis, a major debilitating disease whose predominant feature is the loss of bone *mass* without a reduction in bone *volume* (by decreased density and enlargement of bone spaces), producing porosity and fragility. Osteoporosis among post-menopausal women is one of the most common types of bone disorders, affecting an estimated 20 to 25 million women in the United States alone.

A significant feature of post-menopausal osteoporosis is the large and rapid loss of bone mass due to the cessation of estrogen production by the ovaries. Indeed, data clearly support the ability of estrogens to limit the progression of osteoporotic bone loss, and estrogen replacement is a recognized treatment for post-menopausal osteoporosis in the United States and many other countries.

Estrogens, when administered at low levels, have beneficial effects on bone; however, long-term estrogen replacement therapy has been implicated in a variety of disorders, including an increased risk of uterine and breast cancer. These serious side effects cause many women to refuse this treatment. Alternative therapeutic regimens, designed to lessen the cancer risk, such as administering combinations of progestogen and estrogen, cause some patients to experience regular withdrawal bleeding, which is unacceptable to most older women. Concerns over these significant undesirable side effects associated with estrogen replacement therapy, and the limited ability of estrogens to *reverse* existing bone loss, provide a strong impetus for the development of effective alternative therapies for bone loss that do not cause undesirable side effects.

Another approach in osteoporotic therapy is the use of antiestrogens. In general, antiestrogens inhibit (antagonize) the activity of estrogen in the body. Antiestrogens bind to the estrogen receptor, although it is believed that the interaction between antiestrogens and the estrogen receptor involves a different domain of the receptor than that to which estrogen binds. Some antiestrogens, on the other hand, display pharmacological properties that are a mixture of agonist and antagonist properties. In other words, these compounds cause certain effects that mimic estrogen, while antagonizing other effects that are commonly associated with estrogen administration in cells that express the receptor. Because of this mixed effect of some antiestrogens, they are subject to the same adverse effects associated with estrogen replacement therapy.

One antiestrogen known to display such a mixed agonist/antagonist effect is tamoxifen, a drug used for the treatment of breast cancer. Tamoxifen acts as an estrogen antagonist in its ability to reduce the growth

of breast tumors, but it also acts as an agonist in its ability to reduce the amount of serum cholesterol in both healthy women and women with breast cancer. Love *et al., Annals Int. Med.*, **115**, 860-864 (1991). Tamoxifen also act to increase bone density in breast cancer patients. Love *et al., N. Eng. J. Med.*, **326**, 852-856 (1991). At least one study has suggested that the increases in bone density possible with tamoxifen appear to be restricted to the lumbar spine, with bone *loss* being reported in the radius in some breast cancer patients treated with tamoxifen. Furthermore, tamoxifen treatment has also been suggested to contribute to weight gain among post-menopausal women. Love *et al., Ann. Int. Med., ibid.*

Improved anti-osteoporotics that achieve increases in bone density without causing negative side effects are clearly needed. Unfortunately, no method currently exists for rapidly and efficiently screening large numbers of compounds to identify those that display the desired anti-osteoporotic effects. Because this screening process comprises the most time-consuming and expensive step in identifying improved anti-osteoporotic compounds, development of a rapid method for testing large numbers of compounds to identify those that are likely to possess anti-osteoporotic effect is highly desirable.

It is well established that estrogens exert their effects by first binding to an estrogen receptor, and then the estrogen/estrogen receptor complex binding to DNA. The hormone/receptor complex modulates gene expression *via* this DNA binding. Kumar, *Cell*, **55**, 145-156 (1988). Antiestrogens also bind to estrogen receptors. Although these antiestrogen/receptor complexes bind to DNA they generally fail to modulate gene expression. Both estradiol/estrogen receptor complexes and hydroxytamixofen/estrogen receptor complexes bind *in vitro* to DNA binding domains called estrogen responsive elements. Kumar, *Cell, ibid.*

The conformation of the ligand/receptor complex is a matter of some debate. However, recent studies have suggested a conformational difference between estrogen receptor bound to estradiol and the same estrogen receptor bound to 4-hydroxytamoxifen or ICI 164,384. Klinge *et al., J. Ster. Biochem. Mol. Biol.*, **43**, 249-262 (1992).

In an effort to rationally address the problem of developing improved anti-osteoporotic agents, researchers have investigated proteins known to play a role in bone maintenance. One protein known to influence bone remodelling and bone turnover is transforming growth factor β (TGFβ). Although commonly referred to as a single compound, TGFβ is actually a family of molecules that now is known to include at least three isoforms: TGFβ-1, TGFβ-2 and TGFβ-3. *See* Arrick *et al., Canc. Res.*, **50**, 299-303 (1990). The present inventor has noted that ovariectomy induces a significant decrease in TGFβ-3 in rat bone (data collected by present inventor is unpublished); others have noted the same type of correlation with respect to levels of TGFβ (isoform not specified). *See* Finkelman *et al., Proc. Nat'l Acad. Sci. USA*, **89**, 12190-12193 (1992). Further, the present inventor has noted that administration of raloxifene, an antiestrogen, to ovariectomized rats restores TGFβ-3 concentrations to levels equal to or higher than that found in control animals. The direct correlation between TGFβ-3 levels and circulating levels of estrogen or antiestrogen, and the finding that TGFβ (isoform not specified) plays a significant role in bone remodelling and turnover, suggest that osteoporosis may result from reduced expression of TGFβ-3 in vivo. *See* Noda *et al., Endocrin.* **12**, 2991-2994 (1989).

The hypothesis that reduced levels of TGFβ-3 may allow bone loss is undermined by the findings that TGFβ has been isolated from a large number of sources and exhibits widely divergent effects. For example, it inhibits the growth of mesenchymal cells and epithelial cells, it induces biosynthesis of proteoglycans, fibronectins, and plasminogen activator, and is chemotactic for fibroblasts, macrophages, and smooth muscle cells. *See*, Flaumenhaft *et al., J. Cell. Bio.*, **120**(4), 995-1002 (1993).

Furthermore, antiestrogens such as tamoxifen or toremifene induce human fetal fibroblasts to secrete TGFβ (without reference to isoform) in the absence of estrogen receptor Colletta *et al., Br. J. Cancer*, **62**, 405-409 (1990). TGFβ has been found to stimulate osteoblastic bone formation and to inhibit osteoclast formation and osteoclast activities. Mundy, "Clinical Application of TGFβ", **Ciba Foundation Symposium No. 157**, 137-151, Wiley, Chichester. TGFβ repressed division of one human endometrial cancer cell line (Ishikawa), but was shown to be mitogenic with respect to another such cell line (HEC-50). Murphy *et al., J. Ster. Biochem. Molec. Bio.*, **41**, 309-314 (1992).

Three months of antiestrogen treatment with tamoxifen has been correlated with induction of extracellular TGFβ-1 in breast cancer biopsies. Butta *et al., Cancer Res.*, **52**, 4261-4264 (1992). Decreased concentrations of TGFβ-1 mRNA were found in one human endometrial cancer cell line (HEC-50) grown in media containing 1% ctFBS (twice charcoal stripped FBS) when such cells were exposed to either estradiol or certain antiestrogens. Gong *et al., Canc. Res.*, **52**, 1704-1709 (1992).

TGFβ-2 mRNA is expressed by the T-47D and MDA-MB-231 cell lines. Treatment of these cell lines with estradiol reduced TGFβ-2 mRNA expression, but tamoxifen did not exhibit the same effect. TGFβ-3 induces mitogenesis, collagen synthesis, and alkaline phosphatase activity in osteoblast enriched bone cell cultures at a three to five fold higher rate than TGFβ-1. Arrick et al., *Canc. Res.*, **50**, 299-303 (1990).

A general review of the properties of TGFβ are described in Sporn *et al., J. Cell Bio.*, **105**, 1039-1045 (1987);

Massague, *Cell*, **49**, 437-438 (1987); and Moses, *Cell*, **63**, 245-247 (1990). These references generally describe the properties exerted by TGFβ in *in vitro* and *in vivo* systems.

The complex pattern of expression described above suggests a unique and complex mechanism of regulation of expression of the various TGFβ isoforms. The promoter regions for each of the genes TGFβ-1, TGFβ-2 and TGFβ-3 have been cloned and described. Kim *et al., J. Biol. Chem.*, **264**, 402-408 (1989); Noma *et al., Growth Factors*, **4**, 247-255 (1991); Lafyatis, *et al., J. Biol. Chem.*, **265**, 19128-19136 (1990).

The promoters for TGFβ-2 and TGFβ-3 have been characterized and have been reported to contain cAMP responsive elements, AP-1 sites, AP-2 sites, and SP-1 sites. Noma *et al.* indicated that the TGFβ-2 promoter activity was dependent upon the region of the promoter investigated and the cell line selected for the induction assay.

At least one method for efficiently screening the biological activity of a large number of compounds is described in International Patent Application No. PCT/US92/00419, which claims methods for transcriptionally regulating the expression of a growth factor. This patent disclosed assays to identify compounds capable of inducing transcription via the promoter regions of the human growth hormone gene, the c-ErbB2 gene, the promoter region of the K-ras sequence, and the early promoter and enhancers of cytomegalovirus. This application is directed primarily towards the problem of determining the regulation of various oncogenes.

To date, identifying compounds that are likely to display an anti-osteoporotic effect has required virtually random investigation of individual compounds on the basis of epidemiological studies, the utility of related chemical compounds in achieving the desired effect, and other time consuming and inefficient methods. Both the delay caused by the current screening methods and the economic costs of such inefficient testing emphasize the need for economical and efficient methods for identifying potential anti-osteoporotic drugs worthy of additional investigation.

## BRIEF DESCRIPTION OF THE DRAWINGS

Figure 1 depicts the promoter region of the TGFβ-1 gene.

Figure 2 depicts the promoter region of the TGFβ-2 gene.

Figure 3 depicts the promoter region of the TGFβ-3 gene.

Figure 4 depicts the relative levels of expression of reporter gene in cells transfected with expression constructs comprising the TGFβ-1 promoter operably linked to the CAT gene, the TGFβ-2 promoter operably linked to the CAT gene, or the TGFβ-3 promoter operably linked to the CAT gene, in the presence of a control compound, estrogen, raloxifene, and tamoxifen. In general, the TGFβ-3 construct containing-cells express the highest levels of reporter gene, followed by those containing the TGFβ-2 construct and the TGFβ-1 construct.

Figure 5 depicts the induction of a reporter gene under the control of either the estrogen responsive element or a portion of the TGFβ-3 promoter in the presence of estrogen, raloxifene, and combinations of estrogen and raloxifene. This figure shows the markedly different patterns of regulation exerted by the two regulatory sequences.

Figure 6 is a bar graph showing the relative level of reporter gene expression in cells transfected with expression constructs containing a portion of the TGFβ-3 promoter sequence and exposed to a control compound, estrogen, raloxifene, and combinations of estrogen and raloxifene in both the presence and absence of estrogen receptor. Estrogen receptor is necessary for induction of expression of the reporter gene by both estrogens and antiestrogens.

Figure 7 depicts the various domains of estrogen receptor (ER) protein expressed by the deletion constructs set forth in Example XI. Additionally, the relative fold induction achievable in cells transfected with the various mutant ERs and an expression construct comprising the TGFβ-3 promoter and the luciferase gene are shown.

Figure 8 depicts the levels of reporter gene expression achievable in cells transfected with an expression construct comprising the TGFβ-3 promoter and the CAT gene in the presence of various concentrations of estradiol, raloxifene, tamoxifen, and ICI 164,384. In general, raloxifene is the most potent inducer at all concentrations, followed by ICI 164,384, tamoxifen. Estrogen is the least potent inducer in this system.

Figure 9 depicts the relative expression of reporter gene in CHO cells transfected with an expression construct comprising a portion of the TGFβ-3 promoter and the luciferase gene in the presence of various concentrations of estradiol and raloxifene. In general, raloxifene is the more potent inducer except at low concentrations.

Figure 10 depicts the relative expression of reporter gene in MCF-7 cells transfected with an expression construct comprising the TGFβ-3 promoter and the luciferase gene in the presence of various concentrations of estradiol and raloxifene. Raloxifene is the more potent inducer at all concentrations.

Figure 11 represents the chemical structures of the compounds evaluated in Example X.

Figure 12 represents the relative level of induction of reporter gene expression in MG63 cells transfected with TGFβ-3 promoter/CAT expression constructs and exposed to various concentrations of the compounds set forth in Example X. Overall, compound 177366 is the most potent inducer, while 98005 shows no induction.

Figure 13 depicts the portions of the TGFβ-3 promoter used to identify the 41 base pair raloxifene responsive element, and depicts the relative induction of reporter gene expression by raloxifene in cells transformed with plasmids comprising the indicated portion of the TGFβ-3 promoter sequence operably linked to a reporter gene. Although the fold induction achievable with the pB-301 construct is highest, presence of the raloxifene responsive element (base pairs +35 - +75) is clearly essential for any significant induction of transcription by raloxifene in these cells.

Figure 14 depicts an analysis of the TGFβ-3 promoter. The major transcriptional start site and a CCCTC-motif are depicted as described in Example XI.

Figure 15 depicts the relative expression of reporter gene in Hep2 cells transfected with an expression construct comprising the LDLR promoter and the luciferase gene in the presence of estrogen receptor and various concentrations of estradiol and raloxifene. Generally, raloxifene is the more potent inducer.

Figure 16 depicts the relative expression of reporter gene in Hep2 cells transfected with an expression construct comprising the LDLR promoter and the luciferase gene in the presence of various concentrations of estradiol and raloxifene and in the absence of estrogen receptor. No induction is exhibited by either compound at concentrations at or below $10^{-6}$ M. High concentrations of raloxifene induce expression somewhat, suggesting an alternate, non-ER dependent induction mechanism at such concentrations.

Figure 17 is a flow diagram showing an example of a sequence of steps that can be carried out according to the teachings of the present invention to evaluate compounds with respect to their ability to induce transcription of reporter genes operably linked to the regulatory control element described herein. It is expected that a correlation will exist between compounds showing the induction profiles described in Example XIII and the ability of such compounds to act as anti-osteoporosis drugs *in vivo.*

According to the present invention, there is provided novel and efficient methods for screening chemical compounds to determine whether these compounds are capable of modulating steroid hormone-responsive gene expression from a promoter comprising a raloxifene responsive element. There is also provided nucleic acids consisting essentially of a nucleotide sequence comprising a raloxifene responsive element isolated from the promoter region of a TGFβ gene, and eukaryotic cells transfected therewith. There is also provided a recombinant expression construct comprising the raloxifene responsive element operably linked to a reporter gene. There is also provided a method for inducing bone formation, a method for treating osteoporosis, and a method for treating bone fractures which comprise administering a compound that, when bound to an estrogen receptor, potently induces transcription from a raloxifene responsive element of a promoter region of a TGFβ gene.

The present invention relates to novel and efficient methods of screening chemical compounds to determine whether those compounds are capable of modulating steroid hormone-responsive gene expression from a mammalian promoter comprising a raloxifene responsive element as discovered and described herein. The invention comprises nucleic acids consisting essentially of the nucleotide sequence of a mammalian promoter comprising such a raloxifene responsive element. In a preferred embodiment, the promoter comprising the raloxifene responsive element is derived from the promoter region of the gene for human TGFβ-3 or TGFβ-2.

The invention further comprises recombinant eukaryotic expression constructs comprising a promoter having a raloxifene responsive element that is operably linked to a reporter gene. In preferred embodiments, the reporter gene is the chloramphenicol acetyltransferase gene or the luciferase gene. Particularly, preferred is the luceferase gene. Cells transfected with such eukaryotic expression constructs, that are capable of expressing the reporter gene when such cells are exposed to raloxifene or other anti-estrogenic compounds, are also provided by the invention.

The present invention further comprises a method for inducing bone formation which comprises administering an effective amount of a compound that, when bound to an estrogen receptor, potently induces transcription from a raloxifene responsive element of a promoter region of a TGFβ-3 gene. The present invention also includes a method for treating osteoporosis which comprises administering an effective amount of a compound that, when bound to an estrogen receptor, potently induces transcription from a raloxifene responsive element of a promoter region of a TGFβ-3 gene. The present invention also provides a method for treating bone fractures which comprises administering an effective amount of a compound that, when bound to an estrogen receptor, potently induces transcription from a raloxifene responsive element of a promoter region of a TGFβ-3 gene.

In the first aspect, the invention provides a nucleic acid consisting essentially of a nucleotide sequence comprising a raloxifene responsive element, where the element is isolated from the promoter region of a mammalian, preferably human, transforming growth factor β gene. In preferred embodiments, the transforming

growth factor β gene is the human TGFβ-2 gene or the human TGFβ-3 gene. In further preferred embodiments of this aspect of the invention, the nucleic acid consists essentially of promoter sequences of the TGFβ-3 gene as described in plasmids pB-301 (containing TGFβ-3 promoter sequences from positions -301 to +110); pB-221 (containing TGFβ-3 promoter sequences from positions -221 to +110); pB-91 (containing TGFβ-3 promoter sequences from positions -91 to +110); pB-60 (containing TGFβ-3 promoter sequence from positions -60 to +110); pB-47 (containing TGFβ-3 promoter sequence from positions -47 to +110) and pB-38 (containing TGFβ-3 promoter sequence from positions -38 to +110), as further described herein.

In a second aspect, the invention provides a recombinant expression construct comprising a nucleic acid consisting essentially of a nucleotide sequence comprising a raloxifene responsive element, where the element is isolated from the promoter region of a mammalian, preferably human, transforming growth factor β gene, operably linked to a reporter gene. In preferred embodiments, the transforming growth factor β gene is the human TGFβ-2 gene or the human TGFβ-3 gene. In preferred embodiments, the reporter gene is the chloramphenicol acetyltransferase gene or the luciferase gene. Particularly, preferred is the luciferase gene. In further preferred embodiments of this aspect of the invention, the nucleic acid comprises a promoter sequence consisting essentially of the promoter sequences of the TGFβ-3 gene comprising the plasmids pB-301 (containing TGFβ-3 promoter sequence from positions -301 to +110); pB-221 (containing TGFβ-3 promoter sequences from positions -221 to +110); pB-91 (containing TGFβ-3 promoter sequences from positions -91 to +110); pB-60 (containing TGFβ-3 promoter sequences from positions -60 to +110); pB-47 (containing TGFβ-3 promoter sequence from positions -47 to +110) and pB-38 (containing TGFβ-3 promoter sequence from positions -38 to +110), as further described herein and operably linked to a reporter gene.

In another aspect, the recombinant expression constructs of the invention are capable of expressing the reporter gene encoded by such a construct in eukaryotic cells transfected with such a construct. In preferred embodiments, such eukaryotic cells additionally express an estrogen receptor protein or mutant derivative thereof. In particularly preferred embodiments, expression of the reporter gene by the recombinant expression constructs of the invention is capable of being induced by treatment of such cells with raloxifene or other anti-estrogenic compounds as defined herein.

A third aspect of the invention provides a eukaryotic cell into which has been introduced a recombinant expression construct of the invention. In a preferred embodiment, the eukaryotic cell is a cell transfected with a recombinant expression construct of the invention. In a preferred embodiment, the eukaryotic cells of the invention express an estrogen receptor protein or mutant derivative thereof. In particularly preferred embodiments, expression of the reporter gene in such cells is capable of being induced by treatment of such cells with raloxifene or other anti-estrogenic compounds as defined herein.

The invention also provides methods for screening a multiplicity of compounds to identify those compounds having potential as anti-osteoporotic agents. In one aspect of this embodiment of the invention is provided a method for screening a multiplicity of compounds to identify compounds having potential as anti-osteoporosis agents. The method provided by this aspect of the invention comprises identifying a compound of the multiplicity that is capable of inducing transcription from a raloxifene-responsive element of a mammalian promoter, is a specific transcription inducer, is not capable of inducing transcription from an estrogen-responsive element of a mammalian promoter, and is an anti-estrogenic or non-estrogenic compound. The method provided by this embodiment further comprises the steps of (a) assaying for the ability of the compound to induce transcription from a raloxifene responsive element of a mammalian promoter; (b) assaying for the inability of the compound to induce transcription from a mammalian promoter not having a raloxifene responsive element; (c) assaying for the inability of the compound to induce transcription from an estrogen responsive promoter; and (d) assaying for the ability of the compound to inhibit estrogen induction of transcription from an estrogen responsive promoter in the presence of estrogen.

In a preferred embodiment, the assay of subpart (a) comprises the step of determining the ability of the compound to induce expression of a reporter gene operably linked to the mammalian promoter comprising a raloxifene responsive element. In another preferred embodiment, the assay of subpart (b) comprises the step of determining the inability of the compound to induce expression of a reporter gene operably linked to the mammalian promoter wherein the promoter is not comprised of a raloxifene-responsive element. Another preferred embodiment of this aspect of the invention provides the assay of subpart (c) comprising the step of determining the inability of the compound to induce expression of a reporter gene operably linked to an estrogen responsive mammalian promoter. In a final preferred embodiment, the invention provides the assay of subpart (d) comprising the step of determining the ability of the compound to inhibit estrogen-dependent induction of expression of a reporter gene operably linked to an estrogen responsive mammalian promoter in the presence of estrogen.

In particularly preferred embodiments of this aspect of the invention, the raloxifene responsive mammalian promoter is isolated from a mammalian, preferably human, transforming growth factor β gene. Most preferred

are embodiments wherein the transforming growth factor β gene is the human TGFβ-2 gene or the human TGFβ-3 gene. In other preferred embodiments, the reporter gene is the chloramphenicol acetyltransferase gene or the luciferase gene. In further preferred embodiments of this aspect of the invention, the raloxifene responsive promoter sequences consisting essentially of the promoter sequences of the TGFβ-3 gene comprising the plasmids pB-301 (containing TGFβ-3 promoter sequences from positions -301 to +110); pB-221 (containing TGFβ-3 promoter sequences from positions -221 to +110); pB-91 (containing TGFβ-3 promoter sequences from positions -91 to +110); pB-60 (containing TGFβ-3 promoter sequences from positions -60 to +110); pB-47 (containing TGFβ-3 promoter sequences from positions -47 to +110) and pB-38 (containing TGFβ-3 promoter sequences from positions -38 to +110), as further described herein and operably linked to a reporter gene.

Specific preferred embodiments of the present invention will become evident from the following more detailed description of certain preferred embodiments and the claims.

This specification contains a number of abbreviations. As used herein, "TGFβ" shall mean transforming growth factor β (without reference to isoform). TGFβ-1, TGFβ-2 and TGFβ-3 shall have the meanings well-established in this art, i.e., to represent the three known isoforms of transforming growth factors β genes. As used herein, the abbreviation "CAT" shall be taken to mean chloramphenicol acetyl CoA transferase. "Estradiol" is an estrogen and, at times, is abbreviated herein as E2. As used herein, the abbreviation "ER" shall mean an estrogen receptor protein.

The term "raloxifene responsive element" as used herein refers to nucleotide sequences of the nucleic acid comprising a mammalian promoter region of the TGFβ gene that are capable of inducing transcription of any structural gene to which the raloxifene responsive element is operably linked in host cells that are exposed to raloxifene and estrogen receptor proteins. Raloxifene responsive elements include, but are not limited to the nucleotide sequences comprising the TGFβ promoter sequences of the plasmids pB-301 (containing TGFβ-3 promoter sequences from positions -301 to +110); pB-221 (containing TGFβ-3 promoter sequences from positions -221 to +110); pB-91 (containing TGFβ-3 promoter sequences from positions -91 to +110); pB-60 (containing TGFβ-3 promoter sequences from positions -60 to +110); pB-47 (containing TGFβ-3 promoter sequences from positions -47 to +110) and pB-38 (containing TGFβ-3 promoter sequences from positions -38 to +110), as further described herein, and nucleic acids having substantially the same biological activity as those nucleic acids. This definition is intended to encompass natural allelic variations in the promoter regions of the TGFβ genes. Isolated raloxifene responsive elements of the present invention may be derived from TGFβ promoters of any mammalian species of origin, but are preferably of human origin.

As used herein, anti-estrogens will be taken to include full and partial antagonists of estrogen. All estradiols used in the Examples described herein are 17β-estradiol.

The term "effective amount" represents an amount of a compound that, when bound to an estrogen receptor, is capable of inducing transcription from a raloxifene responsive element when administered to a mammal. The particular dose of compound administered will be determined by the particular circumstances surrounding the case, including the compound administered, the route of administration, the particular condition being treated, and similar considerations.

The term "potently" represents a compound that, when bound to an estrogen receptor, induces transcription from a raloxifene responsive element at a minimum effective concentration (MEC) of less than or equal to 10nM ($1x10^{-8}$M), when the compound is tested in the *in vitro* assay described herein. *See* Examples V, VI, X, and XIV.

All portions of promoter sequences are identified in terms of their distance, in number of nucleotides, from the major transcriptional start site of the gene, taking this start site to be +1 as shown in Figures 1-3. A negative sign (-) preceding the number indicates the nucleotide is 5' to the start site, a positive sign (+) preceding the number indicates the nucleotide is 3' to the start site. The sequences are also identified by the numbering indicated in SEQ ID NOS:1-3, and are specifically correlated with numbering of Figures 1-3.

DNA that encodes the raloxifene responsive elements of the present invention may be obtained, in view of the instant disclosure, by chemical synthesis, by *in vitro* amplification [including but not limited to the polymerase chain reaction (PCR)], or by combinations of these procedures from naturally-occurring sources, such as cultures of mammalian cells, genomic DNA from such cells, or libraries of such DNA.

The raloxifene responsive elements may be advantageously operably linked to reporter genes and used to either transiently or stably transform appropriate host cells through the use of appropriate vectors, constructs, and means well known in the art, such as DNA mediated gene transfer means including but not limited to transfection, electroporation, and virally-mediated infection. The term "recombinant expression construct" as used herein is intended to mean DNA constructs capable of directing the expression of reporter genes to which the raloxifene responsive elements of the invention are operably linked.

DNA regions are operably linked when they are functionally related to each other. For example, a promoter

is operably linked to a coding sequence if it controls the transcription of the sequence; a ribosome binding site is operably linked to a coding sequence if it is positioned so as to permit translation. Generally, operably linked means contiguous.

Reporter genes are genes that encode structural proteins capable of quantification by standard means such as measuring enzymatic activity, colorimetry, chemiluminescence, the presence of radioactivity in a sample, ELISA, antibody binding, radioimmunoassay or other methods of quantification known to those in the art. The reporter gene used will vary depending upon the host selected and the transformation method chosen. Useful reporter genes include but are not limited to chloramphenicol acetyltransferase, luciferase, β-galactosidase, alkaline phosphatase, or any other quantifiable protein product. In a preferred embodiment of the invention, the reporter gene is luciferase.

Transfected cells are cells that have been transfected with raloxifene responsive element-reporter gene recombinant expression constructs made using recombinant DNA techniques. Cells that have been transfected with recombinant raloxifene responsive element-reporter gene expression constructs that express the estrogen receptor, either as a characteristic of such cells or due to the co-transfection of an estrogen receptor encoding expression construct, will express the gene product of the reporter gene under appropriate circumstances (i.e., exposure to an anti-estrogen or other inducer). For example, a preferred cell line appropriate for use in the present invention, MCF-7, constitutively expresses the estrogen receptor. For such cell lines, transfection with the recombinant raloxifene responsive element-reporter gene expression constructs alone will yield cells appropriate for use in the present invention. Alternatively, MG63 cells express reporter genes in a raloxifene-dependent manner, only upon cotransfection of a raloxifene responsive element-reporter gene expression construct and an estrogen receptor expression construct.

Cultures of cells derived from multicellular organisms are desirable hosts for expression of the raloxifene responsive element-reporter gene expression construct. In principle, any higher eukaryotic cell culture that either naturally expresses the estrogen receptor, or that has been genetically modified to express the estrogen receptor (or part of that receptor) is useable. Mammalian cells are preferred, as illustrated in the Examples. Propagation of such cells in cell culture has become a routine procedure. *See* **Tissue Culture**, Academic Press, Kruse & Patterson, editors (1973). Examples of useful host cell lines are MCF-7, MG63, HeLa, RL95.2, HepG2 and CHO cells (all available from the American Type Culture Collection, Rockville, Maryland). For the purposes of the present invention, use of the MCF-7 cell line is particularly preferred, as this cell line constitutively expresses estrogen receptor.

Host cells that express the estrogen receptor or part of that receptor and contain a raloxifene responsive element-reporter gene expression construct can be used to evaluate compounds for their ability to induce transcription via the raloxifene responsive element as described in the Examples *infra*. In a preferred embodiment of the invention, compounds will be considered to induce transcription via a regulatory element (including but not limited to nucleic acid derived from a TGFβ promoter or deletion construct thereof) if transcription of the reporter gene is increased twofold in the presence of the compound compared with expression in the absence of the compound. In a less preferred embodiment, compounds will be considered to be transcriptional inducers if they induce transcription to a level fifty percent above that of the control. In general, however, induction detectably above background is adequate to show induction by a chemical compound.

In the practice of the aspects of the invention embodying screening methods (*see* Example XIII), use of the plasmid pB-301 is preferred due to the high level of responsiveness to raloxifene exhibited by this plasmid. Other embodiments utilize constructs containing the TGFβ-3 promoter region encompassing positions -38 to +75. In all operative embodiments of the invention, the raloxifene responsive element is operably linked to a reporter gene in a context allowing transcription, as this element is necessary to allow the raloxifene responsive induction described herein.

The order of carrying out the steps of the screening methods of the invention may be varied, and in some instances, some of the steps may be omitted. The Examples that follow are illustrative of specific embodiments of the invention, and various uses thereof. They are set forth for explanatory purposes only, and are not to be taken as limiting the invention.

**EXAMPLE I**

**Construction of Reporter Plasmids**

*A. phTG12, pTGF-1 and pB-499*

As a first step in developing cell lines useful in screening potential anti-osteoporotic agents, a series of reporter plasmids encoding the chloramphenicol acetyltransferase gene (CAT) (Gorman *et al.*, *Molec. Cell.*

*Biol.*, **2**, 1044-1051 (1982)) operably linked to promoter sequences from the TGFβ-1, TGFβ-2 and TGFβ-3 genes were obtained from A. Roberts, National Institutes of Health, Laboratory of Chemoprevention (NIH/NCI, Bethesda, MD). These plasmids were designated phTG12 (TGFβ-1), pTGF-1 (TGFβ-2) and pB-499 (TGFβ-3), respectively. The sequences for each of these promoters can be found in Kim *et al., J. Biol. Chem.*, **264**, 402-408 (1989) (TGFβ-1); Noma *et al., Growth Factor,* **4**, 247-255 (1991) (TGFβ-2); and Lafyatis *et al., J. Biol. Chem.*, **265**, 19128-19136 (1990) (TGFβ-3). The promoter sequence of the TGF_-1 gene has been submitted to GenBank/EMBL Data Bank under accession number J04431. The TGFβ-1, TGFβ-2 and TGFβ-3 promoter sequences are shown in Figures 1, 2 and 3, respectively, and as SEQ ID NOS: 1, 2 and 3, respectively.

Alternatively, CAT-containing reporter plasmids operably linked to each of the TGFβ promoter sequences can be produced by subcloning each TGFβ promoter into a commercially-available CAT construct, *for example*, pCAT-Basic (Promega, Madison, WI), using conventional cloning techniques. *See* **Sambrook, Fritsch, and Maniatis, Molecular Cloning: A Laboratory Manual**, 2d ed., Cold Spring Harbor Press, Cold Spring Harbor, New York (hereinafter **Sambrook et al.**).

In order to identify region(s) of the TGFβ-3 gene promoter responsive to the antiestrogen raloxifene, CAT reporter gene expression directed by constructs containing partial sequences of the TGFβ-3 gene promoter were analyzed. Six TGFβ-3 promoter deletion/CAT reporter constructs were obtained from A. Roberts. Plasmid designations and the extent of the promoter region contained in each of these plasmids are set forth below:

| | | |
|---|---|---|
| pB-301 | -301 to +110 | (corresponding to 1896 to 2306 shown in Figure 3 and SEQ ID NO:3) |
| pB-221 | -221 to +110 | (corresponding to 1976 to 2306 shown in Figure 3 and SEQ ID NO:3) |
| pB-91 | -91 to +110 | (corresponding to 2106 to 2306 shown in Figure 3 and SEQ ID NO:3) |
| pB-60 | -60 to +110 | (corresponding to 2137 to 2306 shown in Figure 3 and SEQ ID NO:3) |
| pB-47 | -47 to +110 | (corresponding to 2150 to 2306 shown in Figure 3 and SEQ ID NO:3) |
| pB-38 | -38 to +110 | (corresponding to 2159 to 2306 shown in Figure 3 and SEQ ID NO:3) |

Two additional TGFβ-3 promoter deletion constructs were constructed as described below. The first of these consisted of human TGFβ-3 promoter region sequences corresponding to positions -38 to +75 in the promoter sequence, corresponding to 2159 to 2271 shown in Figure 3 and SEQ ID NO:3 (*see* Lafyatis *et al., ibid.*). The second promoter deletion construct consisted of human TGFβ-3 promoter region sequences corresponding to positions -38 to +35 in the promoter sequence, corresponding to 2159 to 2231 shown in Figure 3 and SEQ ID NO:3. The well-established practice in this art is to identify all promoter sequences identified with respect to the distance from the transcription start site.

These plasmids were generated as follows. Oligonucleotides corresponding to the extent of the TGFβ-3 promoter sequence desired in each plasmid were synthesized using a DNA/RNA synthesizer (Model 394, Applied Biosystems Inc., Foster City, CA) under β-cyanoethyl phosphoamidite synthesis conditions specified by the manufacturer. Complementary pairs of oligonucleotides for each plasmid construct were synthesized, purified, mixed, and allowed to anneal to form double-stranded DNA corresponding to the appropriate TGFβ-3 promoter sequences using conventional methods (**Sambrook et al.**, *ibid.*). *Hin*dIII and *Xba*I restriction enzyme recognition sites were synthesized as the appropriate overhanging ends at the 5' and 3' ends of the sequences, respectively. Double-stranded promoter sequences were then ligated into the *Hin*dIII/*Xba*I-digested CAT reporter plasmid pB-301 and propagated in bacteria under standard conditions. The reporter plasmid produced in this way that contained the -38 to +75 region of the TGFβ-3 promoter was termed pTGFβ+75CAT, and the plasmid that contained the -38 to +75 region of the TGFβ-3 promoter was termed pTGFβ+35CAT. These plasmids were used in CAT assays as described below in Example V.

*B. Luciferase reporter plasmids containing TGFβ-3 promoter deletion constructs, including control containing no portion of the promoter region: pTGFβ-301LUC, pTGFβ-38LUC, pTGFβ+75LUC, pTGFβ +35LUC and pLUC*

Four plasmids were constructed containing the luciferase gene (REF) expressed under the transcriptional control of TGFβ-3 promoter sequences and varying deletion derivatives thereof. The plasmid pTGF-301LUC was made by digesting pB-301 with *Hin*dIII and thereafter the ends of the *Hin*dIII digestion-generated overhang were blunted by treatment with the Klenow fragment of bacterial DNA polymerase I (Boehringer-Mannheim, Indianapolis, IN). *Xba*I digestion was then performed to liberate the portion of the TGFβ-3 promoter corresponding to positions -301 to +110. This fragment was subcloned into *SmaI*/*XbaI*-digested pSP73 (Promega) to generate the shuttle vector pSPTGFβ-301.

After *in vivo* amplification in bacteria, a preparation of isolated and purified pSPTGFβ-301 was digested with *NdeI* and *HindIII*, and the promoter sequence-containing fragment isolated after separation on a 0.8% agarose gel (BRL-LifeTechnologies, Inc., Gaithersburg, MD). The luciferase-containing construct pLDLRLUC10 (as described in U.S. Patent Application Ser. No. 08/018,985, filed March 3, 1993, and further described in Section C., below) was *NdeI*/*HindIII*-digested and purified after agarose gel electrophoresis. These isolated fragments were then mixed, ligated and used to transform bacteria (**Sambrook et al.**, *ibid.*).

The plasmids pTGFβ-38LUC, pTGFβ+75LUC and pTGFβ+35LUC were made by first excising the TGFβ-3 promoter sequences from pB-38, pTGFβ+75CAT and pTGFβ+35CAT, respectively, by *BamHI*/*XbaI* double digestion. The luciferase-containing plasmid pGL2-Basic (or "pGL2LUC") (Promega) was prepared by *NheI*/*BamHI* digestion, and each of the recombinant plasmids made by ligation of the appropriate TGFβ-3 promoter sequences into the luciferase-containing plasmid. These plasmids were used in luciferase assays as described below in Example VI.

A control plasmid containing the luciferase gene but harboring no portion of the TGFβ-3 gene was constructed by digesting pTGFβ+75LUC plasmid DNA with restriction endonuclease *XbaI* and *HindIII*. Protruding ends were filled by Klenow enzyme reaction in the presence of all four dNTPS under standard conditions (**Sambrook et al.**, *ibid.*). The ends thus blunted were ligated with T4 DNA ligase (Boehringer Mannheim) under manufacturer suggested conditions. The resulting plasmid was designated pLUC.

*C. LDLR Promoter Containing Reporter Plasmid: pLDLRLUC10*

The plasmid pLDLRLUC10 was described in U.S. Patent Appln. Ser. No. 08/018,985, filed March 3, 1993 (hereinafter, the '985 application). The construction of this plasmid is described in detail as follows:

A 1546 base pair sequence of the human LDL receptor promoter was amplified using the polymerase chain reaction.

A reaction mixture containing 20 picomoles each of the synthetic oligonucleotides:

5'-GCGCCATATGAGTCTTAACTGCCAAAAATTCTTATCATCAAT-3'
(SEQ ID NO:4)

and

5'-AAGCAAGCTTTCGCAGCCTCTGCCAGGCAGTGTCCCGACCCGGA-3'
(SEQ ID NO:5)

and 1 μg human genomic DNA purified from the adenocarcinoma cell line P3UCLA, 200 μM each of dATP, dGTP, dCTP and TTP, 2.5 units of *Taq* DNA polymerase, 10mM Tris-HCl pH9.3, 50 mM KCl, 15 mM $MgCl_2$, 0.1% gelatin in a final volume of 100 μL was subjected to 30 cycles consisting of 15 sec at 96°C, 30 sec at 55°C, and 1 min at 72°C. The material was subject to gel electrophoresis on a 1% agarose gel and a 1546 basepair (bp) band isolated and restriction enzyme digested with *HindIII* and *NdeI*. This fragment was ligated into the plasmid pSP72 (Promega), which had previously been digested with *HindIII* and *NdeI*. The resulting vector, pNLDLRP, was digested with *NdeI* and *HindIII*, the material was electrophoresed on a 1% agarose gel and the 1546 base pair LDL receptor sequence reisolated therefrom.

Plasmid vector pSv2 was constructed by digesting plasmid pSv2-globin with *HindIII* and *BglII* then ligating an *NruI*-*XhoI* linker into the vector. Plasmid pSv2 globin is disclosed in U.S. Patent No. 4,775,624, which is incorporated by reference. The linker contained the following sequences:

5'-AGCTTCGCGACTCGAGA-3'
(SEQ ID NO:6) and

5'-GATCTCTCCAGTCGCGA-3'
(SEQ ID NO:7).

The resulting vector was designated pSv2-H NXB because it contained a *Bam*HI site, an *Nru*I site, an *Xho*I site and a *Bgl*II site. The *Hind*III-*Bgl*II fragment of plasmid pAlc4(NRRL B-18783), which contains the firefly luciferase gene (REF), was then ligated into the *Hind*III-*Bgl*II site of plasmid pSv2-HNXB.

The 1546 base pair fragment described above was isolated and cloned into the vector pSv2 containing firefly luciferase reporter gene that had been restriction enzyme digested to completion with NdeI and partially with *Hin*dIII. The resulting vector, pLDLRLUC10 contains the human LDL receptor promoter directing expression of the firefly luciferase gene, an ampicillin resistance marker and an origin of replication.

**EXAMPLE II**

**Human Estrogen Receptor Expression Plasmids**

The estrogen receptor-containing mammalian expression constructs pCMVER and pRSV-ER were obtained from B.S. Katzenellenbogen, Department of Physiology and Biophysics, University of Illinois (Urbana-Champaign, IL). *See* Reese and Katzenellenbogen, *J. Biol. Chem.*, **266**, 10880-10887 (1990). These plasmids were used in expression assays as described below, *for example,* in Example VII.

**EXAMPLE III**

**Construction of An Estrogen Responsive Element/Luciferase Gene-Containing Plasmid**

Complementary oligonucleotides corresponding to the estrogen-responsive element (ERE) from the *Xenopus laevis* vitellogenin *Az* gene promoter, corresponding to positions -341 to -310 [(Metzger *et al.*, *Nature*, **334**, 31-36 (1988)] were designed, synthesized, and annealed to form a double-stranded region that is an estrogen responsive element essentially as described in Example I. A sequence comprising an *Xho*I restriction enzyme recognition site was synthesized to be flanking the ERE sequences, the element having the following nucleotide sequence (shown as SEQ ID NOS:8 and 9, respectively):

5'-TCG-AGA-AAA-GTC-AGG-TCA-CAG-TGA-CCT-GAT-CAA-AC-3'
3'-CT-TTT-CAG-TCC-AGT-GTC-ACT-GGA-CTA-GTT-TGA-GCT-5'

The double-stranded ERE was subcloned into *Xho*I-digested pGL12-Basic (Promega), whereby the luciferase gene was placed under the transcriptional influence of the ERE. This plasmid was designated pGL2ERELUC and used in further experiments as described below (Example VI).

**EXAMPLE IV**

**DNA Transfection**

*A. Cell culture*

Mammalian cells were cultured in media (termed 3:1 media) consisting of Dulbecco's modified Eagle's media and F12 media (mixed in a ratio of 3:1, obtained from GIBCO, Grand Island, NY), without phenol red, containing 10% fetal bovine serum (FBS; Hyclone, Logan, UT). Cells were passaged at four day intervals. One day prior to transfection, cells were trypsinized by incubating them with 1mL of a solution of 0.05% trypsin/5.3mM tetrasodium ethylenediamine tetraacetate (GIBCO) for 5min at room temperature, and then seeded in 3:1 media containing 10% charcoal-stripped FBS (csFBS; Hyclone) at densities of one million cells per 10cm culture dish.

*B. Transient co-transfection of TGFβ constructs and human ER constructs*

Co-transfection experiments were performed in MG63 (human osteosarcoma) cells, using the ProFection Mammalian Transfection System (Promega). Ten μg of TGFβ promoter-containing reporter plasmid DNA and

5 μg of human estrogen receptor (hER)-containing expression plasmid (pRSV-ER or pCMV-ER, obtained from B.S. Katzenellenbogen, Department of Physiology and Biophysics, University of Illinois [see Reese and Katzenellenbogen, *J. Biol. Chem.*, **266**, 10880-10887 (1990)] or pRSV (control) plasmid were mixed, co-precipitated, and transfected into 2-4 million cells in 10 cm culture dishes. After incubating the cells with the DNA precipitate at 37°C for 15h, the DNA precipitate was removed by twice washing the cells with Dulbecco's phosphate buffered saline (D-PBS; GIBCO). The cells were then refed with fresh 3:1 media containing 10% csFBS. Compounds to be evaluated for their ability to modulate reporter gene expression were added at the appropriate times and assayed as described below.

*C. Stable co-transfection of TGFβ constructs and human ER constructs*

MCF-7 cells were used for transfection experiments resulting in stable integration of transfected plasmid sequences into the recipient cell genome. In these experiments, TGFβ promoter containing plasmid DNA was mixed with DNA encoding a selectable marker. For example, 60μg of TGFβ promoter-containing plasmid DNA (pTGFβ-301LUC, pGL2LUC or pGL2ERELUC) were mixed with 60μg of the pSV2HYG-derivative, hygromycin resistance gene-containing plasmid pSV2HYGtB (further described in U.S. Patent Application Ser. No. 07/953,633, filed September 29, 1992, hereby incorporated by reference), and transfected onto two million MCF-7 cells using the ProFection system as described above (Promega). After overnight incubation at 37°C, the precipitate was washed from the cells as described above and the cells then refed with fresh 3:1 media containing 10% FBS and cultured for an additional 48h at 37°C, at which time the cells had typically reached confluency. Cells were trypsinized as described above and each culture dish replated into two 10 cm culture dishes in 3:1 media containing 10% FBS. Hygromycin resistance was selected by culturing the cells in media supplemented with 200μg/mL hygromycin B (Calbiochem-Novabiochem, LaJolla, CA). This selective media was replaced with fresh hygromycin B-supplemented media every 2 days without disturbing the cells for the duration of the selection experiment. Clonal colonies became visible after growth for approximately 14 days in selective media. Such clones were isolated and transferred to individual wells of 24-well cell culture dishes (Flow Laboratories, McLean, VA) using a sterile pipette tip. Such clones were grown and maintained in selective media.

To identify hygromycin-resistant clones that had been successfully co-transfected with luciferase gene-containing plasmid sequences, the polymerase chain reaction (PCR) was used to detect luciferase cDNA-derived DNA sequences in transfectant DNA. Oligonucleotide PCR primers were synthesized corresponding to positions 355-373 (sense primer) and 929-911 (antisense primer) of the luciferase cDNA sequence. PCR was performed using Perkin-Elmer GeneAmp PCR System 9600 for 35 cycles under conditions essentially as described by the manufacturer; each PCR cycle included 45 sec at 94°C, 45 sec at 55°C and two minutes at 72°C.

Luciferase-containing hygromycin-resistant clones were incubated with estrogen or raloxifene as described above, and the effect on expression of reporter genes analyzed using assays for the amount on enzymatic activity present in cell extracts. For luciferase assays, cells were lysed in eukaryotic cell lysis reagent containing 0.1M phosphate buffer (pH7.8)/ 1% TritonX-100 (Boehringer Mannheim)/ 2mM EDTA and 1mM dithiothreitol (DTT, Boehringer Mannheim), and assayed using an optimized unenhanced luciferase assay protocol developed by the Analytical Luminescence Laboratory (San Diego, CA). Light output was measured and recorded using a microtitre plate luminometer (ML3000, Dynatech Laboratories, Chantilly, VA). Clones of such cells stably transfected with TGFβ-reporter gene constructs were then used in new anti-osteoporotic screening assays as described below (Example XIII).

## EXAMPLE V

### Analysis of TGFβ Promoter-Mediated Transcriptional Activation by Estrogen and Antiestrogens

It was known in the prior art that expression of the TGFβ-1, TGFβ-2 and TGFβ-3 genes was differentially inducible using estrogen and tamoxifen. See "Background of the Related Art" above. The extent and pattern of this inducibility was characterized using the TGFβ promoter-containing plasmids described above in a series of *in vitro* expression assays as follows.

Cultures of MG63 cells were transiently co-transfected with pRSVER plasmid and either phTG12, pTGF-1, or pB2-499 using the ProFection system (Promega). For each transfected cell culture, DNA-containing calcium phosphate precipitates were added dropwise to each culture dish and mixed thoroughly in the media. The pH of the media was carefully maintained between pH 7.2 and pH 7.4. Transfected cell cultures were then incubated overnight in a 5% $CO_2$ atmosphere at 37°C. For all cultures, the precipitate was removed after overnight incubation by aspirating the media from the culture dishes, followed by rinsing each dish twice with D-PBS. For each co-transfected cell line, the buffer was replaced with 10 ml fresh medium containing 10% csFBS and one of the following compositions:

a. 10 μl ethanol (hormone vehicle) = control;
b. 10 μl 17β-estradiol (Sigma) ("estradiol") at a concentration of $10^{-4}$M in ethanol;
c. 10 μl raloxifene (Eli Lilly Laboratories) at a concentration of $10^{-4}$M in ethanol;
d. 10 μl Tamoxifen (Sigma) at a concentration of $10^{-4}$M in ethanol.

After 24h, incubation with these hormonal preparations (or the vehicle control), cells were washed twice with D-PBS. The cells were then scraped from the culture dishes using a rubber policeman and 1 ml of D-PBS. Cells were collected by centrifugation at 8,000 rpm for two minutes in a tabletop centrifuge (MicroMax Model, IEC, Newark, NY). The supernatant was removed and the cell pellets were resuspended in 150μL of a 0.25M Tris-HCl solution (pH 7.8). Cells were lysed by three cycles of freezing in a dry ice/ethanol bath and thawing in a water bath at 37°C water bath (for 3 minutes each cycle). Lysed cell preparations were centrifuged at 15,000 rpm for 5 minutes at 4°C to remove cell debris. Supernatants containing the soluble cell lysate were transferred to a new set of tubes for assaying chloramphenicol acetyltransferase (CAT) activity.

Before performing CAT assays on such cell lysates, the protein content of each lysate was first determined using a commercially-available assay (BioRad Laboratories, Richmond, CA). An amount of each cell lysate containing 100μg total protein was then mixed with CAT assay buffer (0.4M Tris-HCl (pH 7.8)/ 0.5mM acetyl-CoA (Boehringer Mannheim)/ 0.1 μCi D-*threo*-(dichloroacetyl-1,2-[$^{14}$C]-chloramphenicol) for 15 hours. After this incubation, reactions were stopped by vigorously extracting the reaction mixture with 900μL ethyl acetate. The organic and aqueous phases were separated by brief centrifugation at 14,000 rpm for 1 minute, and approximately 800μL of the organic phase was transferred to a new set of tubes. Ethyl acetate was evaporated to concentrate the CAT-catalyzed reaction products.

Acetylated and unacetylated chloramphenicol species were resolved by thin layer chromatography using a mixture of 95:5 (v/v) chloroform/methanol as the ascending buffer. Radioactivity from each species so resolved was measured using a Betascope 603 blot analyzer (Betagen, Intelligenetics Inc., Mountain View, CA). The percentage of acetylated counts relative to the total counts was calculated to yield relative CAT activities of each transfectant assayed (all CAT activities expressed herein were calculated on this basis). Each assay was performed in duplicate.

A representation of the results of the above experiment for MG63 transfectant cell lines is shown in Figure 4. The results of a representative experiment are tabulated below:

**TABLE I**

| Promoter | Control | Estradiol | | Raloxifene | | Tamoxifen | |
|---|---|---|---|---|---|---|---|
| | | Act. | Fold Ind.† | Act. | Fold Ind. | Act. | Fold Ind. |
| TGFβ-1 | 6.4 | 4.7 | 0.7 | 5.6 | 0.9 | 6.9 | 1.1 |
| TGFβ-2 | 0.8 | 1.29 | 1.6 | 2.3 | 2.9 | 2.0 | 2.6 |
| TGFβ-3 | 0.7 | 2.1 | 2.8 | 5.2 | 7.3 | 1.9 | 2.6 |

† - Fold induction is calculated based on comparison with control

These experiments demonstrate that transcription of the CAT reporter gene is induced by estrogen and the antiestrogens raloxifene and tamoxifen, with raloxifene displaying a greater potency than estrogen, especially for the TGFβ-3 promoter. In contrast, the TGFβ-1 promoter region used in this experiment (positions -1032 to +727) showed no response to either estradiol or raloxifene.

**EXAMPLE VI**

**Differential Induction of TGFβ-3 Promoter and the ERE of the Vitellogenin Promoter by Estrogen and Raloxifene**

The results obtained in the previous Example demonstrated that the TGFβ-3 gene promoter is transcriptionally responsive to both estrogen and "antiestrogen" compounds such as raloxifene and tamoxifen, and that transcription was induced by raloxifene treatment to a relatively greater degree than the degree of transcriptional induction produced in response to estrogen. This example demonstrates that the gene encoding the *Xenopus* protein vitellogenin responds *in vivo* in exactly the opposite fashion, i.e., transcription of the vitellogenin gene is strongly induced by estrogen and only weakly induced by raloxifene. In addition, raloxifene strongly antagonizes estrogen-induced induction of vitellogenin production when the two compounds are given together. The instant results suggested that the TGF_ promoter sequences directing transcription of the reporter genes in the reporter plasmids assayed above in Example VI contain a novel raloxifene-responsive element, characterized by a unique pattern of estrogen and antiestrogen responsiveness.

This pattern of estrogen and antiestrogen responsiveness was further investigated using the reporter gene assay system described in previous Examples. Cultures of MG63 cells were transiently co-transfected with pB-301 and pRSVER as described in Example IV. Such transiently transfected cultures were tested for transcriptional activation of reporter gene expression by treatment with estradiol and raloxifene at concentrations varying in ten-fold increments from $10^{-9}$M to $10^{-5}$M. The combination of estrogen and raloxifene was also tested by assaying the effects of raloxifene at $10^{-8}$M on reporter gene induction in response to estrogen using the same series of concentrations as with estrogen alone. These assays were performed essentially as in Example V.

Similar assays were performed using cultures of MG63 cells transiently transfected with pGL2ERELUC and pRSVER. In these assays, however, the amount of raloxifene added in combination with estrogen was varied so that the raloxifene concentration was twenty times the concentration of estrogen in the mixture (i.e., $10^{-9}$M estrogen/2 x $10^{-8}$M raloxifene; $10^{-8}$M estrogen/ 2 x $10^{-7}$M raloxifene, etc.).

Transfected cells were treated with varying amounts and combinations of hormones and then rinsed twice with D-PBS. Cells were then lysed upon incubation with 250μL of eukaryotic cell lysis reagent (as described in Example VI) at 4°C for 20 min and transferred to microcentrifuge tubes by scraping with a rubber policeman. Cell lysates were centrifuged at 14,000 rpm for one minute to remove cell debris. Cell extracts (supernatant) were then assayed for protein content and luciferase activities.

Luciferase assays were performed as follows. 50μL of each cell extract was added to 100μL of reagent A buffer (containing 4.0mM ATP/15mM $MgSO_4$/ 30mM tricine buffer (pH7.8)/ 10mM DTT) in individual wells of a microtiter plate. 100μL of 1 mM D(-)-luciferin (in 0.1M $KPO_4$ (pH 7.8); Boehringer Mannheim) were added to each well and the amount of light produced measured by using a ML3000 microtiter plate luminometer (under conditions of integrate flash mode, high gain, integrate window = 10 seconds, at a temperature of 22°C). Luciferase activities were calculated as total light output relative to protein content in each cell lysate sample.

The results of these tests are set forth in tabular form below:

TABLE II

For pB-301

| | Control | $10^{-9}$M | $10^{-8}$M | $10^{-7}$M | $10^{-6}$M | $10^{-5}$M |
|---|---|---|---|---|---|---|
| Raloxifene | 0.7 | 2.4 | 9.6 | 9.63 | 9.05 | 5.25 |
| Estradiol | 0.7 | 1.1 | 3.2 | 5.5 | 5.2 | 8.0 |
| E2 + Ral | --- | 17.3 | 16.0 | 7.3 | 9.3 | 10.5 |

For pGL2ERELUC:

| | Control | $10^{-9}$M | $10^{-8}$M | $10^{-7}$M | $10^{-6}$M | $10^{-5}$M |
|---|---|---|---|---|---|---|
| Raloxifene | 1.7 | 1.9 | 1.5 | 2.0 | 1.9 | 1.9 |
| Estradiol | 1.7 | 5.8 | 5.5 | 5.9 | 6.2 | 6.5 |
| E2 + Ral | --- | 1.8 | 1.9 | 2.1 | 2.3 | 2.4 |

Representative results of these experiments are shown in Figure 5.

These results clearly demonstrate the existence of a distinct promoter region in the TGFβ gene promoter that is responsive to antiestrogens. While raloxifene acts as a potent antagonist to transcription initiated by ERE-containing promoters of genes such as the vitellogenin gene, it was found herein to act as a super-agonist in inducing transcription from the TGFβ-3 promoter. Interestingly, at low concentrations, raloxifene and estrogen *synergistically* induced transcription from the TGFβ-3 promoter in the reporter plasmids of the invention, suggesting that raloxifene-induced gene transcription may be mediated by a novel mechanism.

## EXAMPLE VII

### Estrogen Receptor Dependent Gene Activation of TGFβ-3 by Estrogen and Antiestrogens

It was known in the prior art that the ability of both estrogens and antiestrogens to influence TGFβ production is dependent on the expression of estrogen receptor (ER), but the level at which this influence is exerted was not known (i.e. transcriptional, translational or post-translational). A series of experiments were therefore performed to investigate the putative dependence on ER expression of induction of reporter gene expression using the TGFβ promoter-containing constructs of the invention. Lack of ER expression virtually abolishes expression of TGFβ-3, regardless of the presence of estradiol or raloxifene. Through the use of mutant ER proteins, it has been determined that different domains of the ER molecule are responsible for estrogen and raloxifene induction.

*A. ER dependent induction of TGFβ-3*

Cultures of MG63 were prepared for co-transfection as in Example IV. One such culture was co-transfected with pB2-499 and pRSVER and another was co-transfected with pB2-499 and pRSV vector plasmid (control). The ability of the following compounds to induce transcription via the raloxifene responsive element of the TGFβ-3 gene was then assayed essentially as described in Example V:

(a) ethanol
(b) 17β-estradiol ($10^{-7}$M)
(c) raloxifene (2 x $10^{-6}$M)
(d) 17β-estradiol ($10^{-7}$M) and raloxifene (2 x $10^{-6}$M)

The results of one such experiment are set forth in the following Table, and a representative example of a thin-layer chromatogram produced thereby is shown in Figure 6.

**TABLE III**

| | Hormone Vehicle | Estradiol | Raloxifene | Estradiol + Raloxifene |
|---|---|---|---|---|
| Control Plasmid | 0.9 | 1.0 | 1.1 | 1.2 |
| ER Expression Plasmid | 1.3 | 4.6 | 45.7 | 33.7 |

These results clearly indicate that ER expression is required for TGFβ-3 gene promoter-mediated induction of reporter gene expression in response to estrogen, antiestrogens and combinations thereof.

*B. Analysis of ER protein domains required for induction from the TGFβ-3 promoter*

It was disclosed in the prior art that the ER protein region designated "E" is necessary for estrogen binding, while region "C" is necessary for DNA binding. *See* Kumar *et al., EMBO J.*, **9**, 2231-2236 (1986). It has also been well established that the "C" region is essential for ER activation of ERE-containing genes, while the "E" region is required for estrogen-dependent inducibility.

To determine the regions of the ER involved in induction of transcription from the TGFβ-3 promoter, cultures of MG63 cells were prepared for co-transfection as in Example IV. Cells were transfected with mixtures of pTGFβ-301LUC and one of the following expression plasmids comprising various deletion mutants of ER:

a. pCMV-ER
b. pCMV-ERΔA/B
c. pCMV-ERΔB/C/D
d. pCMV-ERΔE/F
e. pCMV-$ER_{1-530}$
f. pCMV-$ER_{L540}Q$

*See* Reese & Katzenellenbogen, *J. Biol. Chem.*, **266**, 10880-10887 (1990) and Figure 7 for a further explanation of the extent of each deletion in these plasmids.

The ability of these mutant ERs to mediate raloxifene-induced TGFβ-3 activation was tested by treating co-transfected cells with vehicle (10μL ethanol) or $10^{-7}$M raloxifene. The increase of raloxifene-induced luciferase activity over basal activity was calculated as the fold induction by raloxifene in the presence of different mutant ER forms as depicted in Figure 7.

The results of one such experiment are shown in the following Table:

**TABLE IV**

| ER mutant form | vehicle | raloxifene | fold induction |
|---|---|---|---|
| pCMV-$ER_{wt}$ | 3.7 | 32.8 | 8.9 |
| pCMV-ERΔA/B | 14.2 | 39.4 | 2.8 |
| pCMV-ERΔB/C/D | 21.2 | 73.4 | 3.4 |
| pCMV-ERΔE/F | 17.4 | 20.9 | 1.2 |
| pCMV-$ER_{1-530}$ | 13.4 | 26.5 | 2.0 |
| pCMV-$ER_{L540^Q}$ | 12.2 | 39.8 | 3.3 |

These results show that the hormone binding domain (i.e., the "E" region of the estrogen receptor molecule) is both necessary and sufficient to mediate raloxifene-stimulated, TGFβ-3 promoter-mediated transcription of reporter genes in the reporter plasmids of the invention. The "C" region of the ER molecule appears not to be required for this process. This finding further supports the suggestion that a novel mechanism of activating gene transcription involving ER may be involved in transcription from the TGFβ promoter.

**EXAMPLE VIII**

**Activities of Estrogen and Antiestrogens on TGFβ-3 Promoter**

Transcriptional activation of TGFβ promoter-mediated gene expression by estrogen and antiestrogen compounds was found to be concentration-dependent. Cultures of MG63 cells were transiently co-transfected with pB-301 and pRSVER as described in Example V. Such transiently transfected cell cultures were divided into four groups of twelve cultures, and each of the four groups was used to test the ability of one estrogen or antiestrogen compound to induce transcription from the TGFβ-3 promoter individually. For each group of twelve cultures, the particular estrogen or antiestrogen compound was tested in replicate cultures at six concentrations, varying in tenfold increments from $10^{-9}$M to $10^{-5}$M, as well as one set of replicate cultures treated with vehicle only (for a total of twelve cultures per experimental treatment). Hormones were dissolved in ethanol and applied to the cultures in media as described above. The four estrogens and antiestrogens tested were:

a. 17β-estradiol (Sigma Chemical Corp., St. Louis, MO);
b. raloxifene (Eli Lilly, Indianapolis, IN);
c. tamoxifen (Sigma);
d. ICI 164,384 (described in European Patent No. EP138504, issued 20 July 1988).

After 24 hours of hormone treatment (or control) the cells were washed, harvested, lysed, and assayed for CAT activity as described in Example V. The results of one such experiment are tabulated below and are depicted in Figure 8:

**TABLE V**

| | vehicle | $10^{-9}$M | $10^{-8}$M | $10^{-7}$M | $10^{-6}$M | $10^{-5}$M |
|---|---|---|---|---|---|---|
| estradiol | 0.85 | 0.96 | 1.1 | 1.6 | 4.3 | 3.4 |
| raloxifene | 0.85 | 6.9 | 19.2 | 18.1 | 19.6 | 14.8 |
| tamoxifen | 0.85 | 0.91 | 1.2 | 0.98 | 2.9 | 13.5 |
| ICI 164,384 | 0.85 | 0.89 | 0.88 | 10.9 | 15.2 | 6.5 |

Although all estrogens and antiestrogens influence TGFβ-3 promoter activity, each compound exhibits its own distinctive dose-response curve. Raloxifene is by far the more potent activator, displaying more than a 20-fold induction of reporter gene transcription and having an $ED_{50}$ at nanomolar concentrations. In contrast, estradiol showed only a 4-fold induction of reporter gene expression and an $ED_{50}$ that was two orders of magnitude higher than that of raloxifene. Tamoxifen activates the TGFβ-3 promoter only at high levels (i.e., greater than micromolar). ICI 164,384 showed an $ED_{50}$ of $10^{-7}$M, but this compound appears to be much less active at high concentrations ($10^{-5}$M). These results demonstrate that a novel element has been found in the promoter region of the TGFβ-3 gene termed a raloxifene responsive element (RRE). This element induces transcription in the presence of both estrogens and antiestrogens and each of these compounds exhibits a characteristic dose-response profile of transcriptional activation.

**EXAMPLE IX**

**Raloxifene-Mediated Transcriptional Activation of the TGFβ-3 Promoter in CHO and MCF-7 Cells**

The ability of raloxifene to induce transcription from the TGFβ-3 promoter distinct from estrogen-mediated induction was demonstrated in a variety of cell lines.

*A. TGFβ-3 activation in CHO cells*

Cultures of CHO cells were transiently co-transfected with pB-301 and pRSVER as described in Example IV and were used to determine the ability of both raloxifene and estradiol to induce transcription via the raloxifene responsive element. Twelve transiently transfected cultures were treated in replicate with either estradiol or raloxifene at six concentrations varying in tenfold increments from $10^{-9}$M to $10^{-5}$M (as well as a vehicle only control for a total of twelve cultures). Hormones were dissolved in ethanol and applied to the cultures in media as described above.

After 24 hours of incubation with the hormonal preparations (or the control), the cells were washed, harvested, lysed, and assayed for CAT activity as described in Example V. The results are tabulated below and depicted in Figure 9:

**TABLE VI**

| | 0M | $10^{-9}$M | $10^{-8}$M | $10^{-7}$M | $10^{-6}$M | $10^{-5}$M |
|---|---|---|---|---|---|---|
| estradiol | 10.6 | 11.9 | 22.6 | 29.4 | 32.9 | 33.3 |
| raloxifene | 10.6 | 21.3 | 21.2 | 19.7 | 20.8 | 10.1 |

These results demonstrated that the previously-observed responsiveness of the TGFβ-3 promoter to estrogen and raloxifene was retained when assayed in CHO cells.

*B. TGFβ-3 activation in MCF-7 cells*

Cultures of MCF-7 cells were transiently transfected with pTGFβ-301LUC as described in Example IV. These cultures were used to test the ability of raloxifene and estradiol to induce transcription in this cell type. Cultures were treated with either estradiol or raloxifene at one of the following six concentrations: 0M, $10^{-9}$M, $10^{-8}$M, $10^{-7}$M, $10^{-6}$M and $10^{-5}$M. Hormones were dissolved in ethanol and applied to the cultures in media as described above.

After 24 hours of treatment with the hormonal preparation (or the vehicle control), the cells were washed, harvested, lysed, and assayed for LUC activity according to the method of Example VI. The results of this experiment are tabulated below, and the results from a series of such experiments are summarized in Figure 10:

**TABLE VII**

| | 0M | $10^{-9}$M | $10^{-8}$M | $10^{-7}$M | $10^{-6}$M | $10^{-5}$M |
|---|---|---|---|---|---|---|
| estradiol | 7.8 | 19.2 | 9.1 | 107.3 | 101.3 | 122 |
| raloxifene | 7.8 | 158 | 309 | 314 | 451 | 206 |

(Luciferase activity expressed in thousands of units)

Similar assays were performed in human endometrial cancer cells (RL59.2), human cervical cancer cells (HeLa), and monkey kidney cells (COS-1) (American Type Culture Collection, Rockville, MD). Transcription initiated by the TGFβ-3 promoter was found to be induced by estrogen and raloxifene in all cell types tested (with variations in the magnitude of induction). These results demonstrate that estrogen and raloxifene-mediated induction of reporter gene transcription from the TGFβ-3 promoter is not restricted to specific cell types. The different levels of induction in different cells, however, might indicate the abundance of other factors in these cells involved in regulation. The fact that raloxifene and estrogen responsiveness of the TGFβ-3 promoter were found using both luciferase and CAT as reporter genes indicates that this regulation is a general characteristic of gene expression from this promoter.

## EXAMPLE X

### Comparative Induction of Reporter Gene Expression from the TGFβ-3 Promoter by Raloxifene and Related Compounds

Antiestrogen compounds were known in the prior art to be capable of inducing TGFβ gene expression in a dose-dependent manner. Knabbe *et al., Am. J. Clin. Onc.*, **14** (Suppl.2), S15-S20 (1991). Furthermore, as shown in Example VIII above, raloxifene and tamoxifen were found to be capable of inducing TGFβ-3 gene expression in a dose dependent manner.

The experiments described in this Example were performed in order to correlate the ability of compounds to induce transcription via the raloxifene responsive element of the TGFβ-3 promoter with their known uterotropic capacities as demonstrated in ovariectomized rats. Seven compounds that are structurally related to raloxifene were tested for their ability to induce transcription via the raloxifene responsive element of the TGFβ-3 promoter. These compounds can be distinguished on the basis of a spectrum of in vivo activity ranging from uterotropic (LY112676, LY81099, and LY13314) to anti-uterotropic (LY113526, LY139482 and LY177366), and included a compound known to be inert *in vivo* (LY98005).

The IUPAC names for the compounds and the U.S. Patents in which they have been claimed are as follows:

| | | |
|---|---|---|
| 113526 | 2-(p-hydroxyphenyl)benzo[B]thien-3-YL p-[2-(1-pyrrolidinyl)ethoxy] phenyl ketone | 4,133,814 |
| 139482 | [4-[2-(hexahydro-1H-azepin-1-yl) ethoxy]phenyl][6-hydroxy-2-(4-hydroxyphenyl)benzo[B]thien-3-yl] methanone | 4,380,635 |
| 177366 | [6-(2,2-dimethyl-1-oxopropoxy)-2-[4-(2,2-dimethyl-1-oxopropoxy) phenyl]benzo[B]thien-3-yl][4-[2-1-piperidinyl)ethoxy]phenyl] methanone hydrochloride | 07/902,933, filed July 28, 1992, incorporated by reference) |

| | | |
|---|---|---|
| 98005 | p-hydroxyphenyl 3-(-hydroxyphenyl)benzo[B]thien-2-yl ketone | |
| 112676 | (p-hydroxyphenyl) 5-hydroxy-3-phenylbenzo[B] thien-2-yl ketone | 4,075,227 |
| 81099 | p-hydroxyphenyl 3-phenylbenzo[B] thien-2-yl ketone | 4,075,227 |
| 133314 | [3,4-dihydro-2-(4-methoxyphenyl)-1-naphthanlenyl] [4-[2-(1-pyrrolidinyl)ethoxy]phenyl] methanone, methanesulfonic acid salt | |

These compounds are depicted in Figure 11.

Raloxifene, LY81099, LY98005, LY112676, LY113526, LY13314, LY139482, and LY177366 (Eli Lilly and Company, Indianapolis, Indiana) were assayed to compare their ability to induce transcription from the promoter of the TGFβ-3 gene at varying concentrations. Cultures of MG63 cells transiently co-transfected with pB-301 and pRSVER were treated as in Example VI with the above compounds at concentrations of 0M, $10^{-9}$M, $10^{-8}$M, $10^{-7}$M, $10^{-6}$M and $10^{-5}$M. The experimental results are shown in tabular form and depicted in Figure 12.

TABLE VIII

| | 0M | $10^{-9}$M | $10^{-8}$M | $10^{-7}$M | $10^{-6}$M | $10^{-5}$M |
|---|---|---|---|---|---|---|
| raloxifene | 0.61 | 2.1 | 6.4 | 7.5 | 7.0 | 4.0 |
| LY81099 | 0.61 | 0.6 | 0.5 | 0.7 | 0.9 | 2.3 |
| LY98005 | 0.61 | 0.4 | 0.5 | 0.5 | 0.4 | 0.5 |
| LY112676 | 0.61 | 0.6 | 0.6 | 0.5 | 1.7 | 3.8 |
| LY113526 | 0.61 | 0.7 | 0.7 | 0.6 | 2.0 | 2.1 |
| LY133314 | 0.61 | 0.7 | 0.7 | 3.1 | 6.6 | 1.0 |
| LY139482 | 0.61 | 0.8 | 2.5 | 2.7 | 2.5 | 2.0 |
| LY177366 | 0.61 | 1.1 | 7.9 | 10.2 | 7.9 | 6.1 |

Compounds that displayed uterotropic properties in *vivo* (i.e., estrogenic compounds) showed $ED_{50}$ values of approximately $10^{-7}$M and had a relatively lower-fold induction of reporter gene expression from the TGFβ-3 promoter, much like the profile exhibited by estrogen in Example VIII. In contrast, compounds that have a profile similar to raloxifene *in vivo* demonstrate an $ED_{50}$ of $10^{-9}$M and a relatively greater fold induction than estrogenic compounds. *In vivo* data regarding raloxifene was set forth in U.S. Patent Appln. Ser. No. 07/920,933, filed July 28, 1992, and is incorporated by reference. In summary, compounds exerting estrogen like qualities *in vivo* show significantly less induction of transcription via the TGFβ-3 promoter than compounds that exerted antiestrogen-like qualities *in vivo*.

The results of these experiments demonstrate the utility of the reporter gene-containing expression plasmids described herein as a screening technique for identifying potential anti-osteoporosis agents, because those compounds that are raloxifene-like in their induction profiles and $ED_{50}$'s show relatively lower uterotropic effects than estrogen-like compounds having lower induction profiles and higher $ED_{50}$'s in the present assay.

**EXAMPLE XI**

**Localization of the Raloxifene Response Element in TGFβ-3 Promoter in the Region from +35 to +75**

At least a portion of the raloxifene response element (RRE) in the human TGFβ-3 gene promoter was approximately localized to a particular 41 nucleotide sequence found at positions +35 through +75. This sequence was found to be necessary for mediating raloxifene-induced transcriptional activation of reporter gene expression in the TGFβ-reporter gene expression constructs described above.

*A. Identification of a raloxifene responsive element by functional analysis of TGFβ-3 promoter deletion mutants prepared in vitro*

Cultures of MG63 cells transiently co-transfected with pCMVER and one of a variety of TGFβ-3 promoter deletion reporter constructs (including pB-499, pB-301, pB-221, pB-91, pB-60, pB-47, pTGFβ-38LUC, pTGFβ+75LUC, pTGFβ+35LUC and pLUC) were generated as described in Example I. These cultures were then treated with either ethanol (as a control) or $10^{-6}$M raloxifene. The degree of induction of reporter gene expression after treatment with raloxifene relative to that obtained by treatment with vehicle alone was calculated for each TGFβ-3 promoter deletion construct and are tabulated below and are depicted in Figure 13:

TABLE IX

| Vector Plasmid | TGFβ-3 Promoter region | Fold induction by raloxifene |
|---|---|---|
| pB-499 | -499 - +110 | 6.8 |
| pB-301 | -301 - +110 | 13.1 |
| pB-221 | -221 - +110 | 8.7 |
| pB-91 | -91 - +110 | 10.1 |
| pB-60 | -60 - +110 | 12.5 |
| pB-47 | -47 - +110 | 11.5 |
| TGFβ-38LUC | -38 - +110 | 7.1 |
| TGFβ+75LUC | -38 - +75 | 5.8 |
| TGFβ+35LUC | -38 - +35 | 1.2 |
| pLUC vector alone | | 0.5 |

These results localize at least one portion of the raloxifene responsive element to positions +35 to +75 in the TGFβ-3 promoter sequence.

*B. Nucleotide sequence of the raloxifene responsive element*

The nucleotide sequence of the TGFβ-3 promoter from position -38 to +110 was depicted in Figure 14. The raloxifene responsive sequence was found above to be the sequence depicted in the Figure in outline form. The open arrow indicates the major transcription start site (+1). The two black arrows indicate the two minor transcription start sites. The "TATA" sequence is shown in the open box. A putative CCCTC-motif is indicated by a series of horizontal arrow heads under the sequence of the putative raloxifene responsive element. *See* Lobanenekov *et al. Oncogene*, **5**, 1743-1753 (1990).

Two conclusions can be drawn from the TGFβ-3 analysis. The first is that no palindromic sequences homologous to the ERE was found in this region of the TGFβ-3 promoter. This finding is consistent with the results shown in Example VII which demonstrated that DNA binding activity of ER is not required. Second, ER-mediated raloxifene activation of TGFβ-3 most likely requires other factors that are capable of binding to the raloxifene responsive sequence. A good candidate for such a protein is the CTCF factor identified by Lobanenkov *et a*l. which is involved in *c-myc* gene regulation. These findings may lead to the identification of other genes as potential raloxifene inducible genes that have raloxifene responsive elements in their promoters. Furthermore, such genes could be used to identify genetic elements having the activity of raloxifene responsive elements for use in the screening procedure set forth in Example XIII.

The raloxifene responsive element of the present invention was used to search the GenBank sequence library; significant homology was found between this element and elements in the following genes:

| GenBank/EMBL Data Bank | |
|---|---|
| Accession No.: | Gene: |
| X56595 | Chicken type VI collagen _-2 |
| X55373 | Human ETS-2 promoter region |
| M30137 | Human ETS-2 (5' flank) |
| D10231 | Mouse glucose transporter (enhancer 2) |
| M12731 | Mouse N-myc proto-oncogene |
| M13945 | Mouse pim-1 proto-oncogene |
| X63281 | *R. norvegicus* N-myc gene |
| X16995 | Mouse N10 gene |
| M94152 | Rat adenosine receptor |
| M20131 | Rat cytochrome P450IIE1 |
| M34111 | Rat PTHrP |
| J05097 | Rat substance P receptor |
| M64236 | Rat substance P receptor |

This finding supports the existence of this element as a discrete and important regulatory unit capable of mediating pleiotropic physiological effects *in vivo* in a variety of tissues and cell types.

## EXAMPLE XII

### Estrogen and Raloxifene Induce LDL Receptor Promoter Activation

LDL receptor expression plays an essential role in regulation of serum LDL-cholesterol uptake. It has been known that estrogen induces LDL receptor messenger RNA *in vivo*. Ma *et al., Proc. Natl. Acad. Sci. USA*, **83**, 792-796 (1986). As shown in this Example, this activation of LDL receptor promoter sequence by estrogen is mediated by ER. Raloxifene also induces LDL receptor promoter; however, this induction is ER independent.

*A. Estrogen and antiestrogen induced LDLR-Luc production in presence of ER*

ATCC strain HepG2 cells were co-transfected with pLDLRLUC10 and pRSVER as described in Example IV. These cells were exposed to estradiol and raloxifene under the conditions set forth in Example VI. The results are tabulated below and a series of experiments are depicted in Figure 15:

**TABLE X**

| | 0M | $10^{-9}$M | $10^{-8}$M | $10^{-7}$M | $10^{-6}$M | $10^{-5}$M |
|---|---|---|---|---|---|---|
| estradiol | 20.9 | 16.6 | 15.4 | 40.7 | 74.0 | 69.0 |
| raloxifene | 20.9 | 20.9 | 19.9 | 26.9 | 14.6 | 14.6 |

*B. Estrogen and antiestrogen-induced LDLR-Luc production in the Absence of ER*

ATCC strain HepG2 cells were co-transfected with pLDLRLUC10 and pRSV vector plasmid as described in Example IV. These cells were exposed to estradiol and raloxifene under the conditions set forth in Example VI. The results are tabulated below and a representative series of such experiments are set forth in Figure 16:

TABLE XI

| | 0M | $10^{-9}$M | $10^{-8}$M | $10^{-7}$M | $10^{-6}$M | $10^{-5}$M |
|---|---|---|---|---|---|---|
| estradiol | 1597 | 1645 | 1792 | 1574 | 1578 | 2445 |
| raloxifene | 1597 | 1652 | 1234 | 1025 | 1291 | 5561 |

These results show that both raloxifene and estrogen have the ability to induce LDL receptor expression. This result provides an explanation of serum lipid lowering effect by estrogen and raloxifene *in vivo* in both animal models and humans.

**EXAMPLE XIII**

**Method for Screening Potential Anti-Osteoporosis Agents**

Based on the foregoing Examples, an assay using luciferase as a reporter gene was designed to screen for potential anti-osteoporosis agents.

Cultures of MCF-7 cells were stably transfected with:

1. pTGFβ-301LUC;
2. pGL2LUC; or
3. pGL2ERELUC;

using the methods described in Example IV. The cells are then used in the inventive method depicted in Figure 17.

STEP 1. The first procedure that is used in the screening assay is to determine the ability of a compound to induce transcription from the TGFβ-3 promoter. The assay is performed essentially as described in Example IX, using MCF-7 cells stably transfected with pTGFβ-301LUC. Cell culture and assay conditions are adapted to the 96-well microtiter plate format. Cells are seeded in a 96-well plate at a density resulting in approximately 50% confluency. Test compounds may be selected from a variety of sources, including pharmaceutical research records, chemical manufacturers products lists, and naturally-occurring sources such as fermentation extracts. Cells are incubated in growth media (as described in Example IV) containing the test compound for about 24 hours. Cells are then lysed *in situ* on the plate, and the lysates subjected to both a quantitative protein assay and to the luciferase activity assay. Compounds that induce a greater than two-fold increase in luciferase activity are considered competent for further testing.

STEP 2. Assays are performed with compounds identified as described in Step 1 on cell cultures that have been stably transfected with pGL2LUC to determine whether such compounds are general transcription inducers. As such general transcriptional inducers lack the transcriptional induction specificity required for potential anti-osteoporetics that are modulators of raloxifene-responsive element-dependent gene expression, such general inducers are excluded from further testing.

STEP 3. Compounds having the required transcriptional induction specificity (that is, are capable of inducing transcription induction in pTGFβ-301LUC cells without inducing transcription in cells transfected with pGL2LUC) for potential anti-osteoporetics, that are modulators of raloxifene-responsive element-dependent gene expression, are then assayed to determine whether such compounds induce transcription from an estrogen responsive element. Cells stably transfected with pGL2ERELUC are assayed as described in Example VI both in the presence and in the absence of estradiol. Compounds that activate pGL2ERELUC in the *absence* of estrogen are disqualified for further testing, because the capacity of these compounds to induce transcription from an estrogen-responsive element in the absence of estrogen evidences potential estrogenic activity *in vivo*.

STEP 4. Compounds that have fulfilled the criteria of Steps 1 through 3 are then further tested to determine whether such compounds are either anti-estrogenic or non-estrogenic/non-antiestrogenic. To this end, the compounds are assayed in the presence of estradiol in cells stably transfected with pGL2ERELUC. Inhibition of estrogen-induced luciferase activity in this assay indicates that such compounds have anti-estrogenic activity. Both anti-estrogenic and non-estrogenic compounds will be characterized for their dose-response profiles and $ED_{50}$ values. Further experiments may be done to establish the dose-response profiles of such compounds and to compare them with known anti-estrogens like raloxifene. *See* Example X.

Following this screening protocol, conventional assays, particularly an *in vivo* assay involving appropriate animal model systems, may be used to further characterize the anti-estrogenic properties of the compounds identified as described herein. Development of such anti-estrogenic compounds having desirable anti-osteoporotic properties may then be advantageously and expeditiously achieved from the compounds identified in this assay.

**EXAMPLE XIV**

**Correlation between *in vitro* and *in vivo* activity**

The following experiments were performed to demonstrate the correlation between the *in vitro* assay and an *in vivo* model of post-menopausal osteoporosis. The *in vitro* assay measures the test compound's ability to induce transcription via the raloxifene responsive element of the TGFβ-3 promoter. The *in vivo* model measures the changes in bone (femur) mineral density in ovariectomized rats. The following compounds were used in these experiments:

| | |
|---|---|
| raloxifene | 6-hydroxy-2-(4-hydroxyphenyl)-3-[4-(2-piperidinoethoxy)benzoyl]benzo[b]thiophene |
| 088074 | 6-hydroxy-2-(4-hydroxyphenyl)-3-(4-hydroxybenzoyl)benzo[b]thiophene |
| 156678 | 6-hydroxy-2-(4-hydroxyphenyl)-3-[4-(2-(3-methylpyrrolidine)ethoxyl)benzoyl]benzo[b]thiophene |
| 171147 | 2-(4-hydroxyphenyl)-3-[4-(2-piperidinoethoxy)benzoyl]benzo[b]thiophene |
| 309503 | 6-hydroxy-2-(4-hydroxyphenyl)-3-[4-(2-hydroxyethoxy)benzoyl]benzo[b]thiophene |

These compounds are prepared as generally described in U.S. Patent No. 4,133,814 (issued January 9, 1979) which is incorporated herein by reference.

Raloxifene, 088074, 156678, 171147, and 309503 (Eli Lilly and Company) were assayed to determine their ability to produce a two-fold induction in the transcription from the promoter of the TGFβ-3 gene, as measured by luciferase activity, at a concentration of less than 10 nM [i.e. minimum effective concentration (MEC) < 10 nM]. One day prior to transfection, HeLa cells were trypsinized with a solution of 0.05% trypsin/5.3mM tetrasodium ethylenediamine tetraacetate (EDTA) to achieve a single cell suspension. The detached confluent cells were counted and diluted to a concentration of 1,000,000 cells/mL. Three million cells and 3:1 media (25mL) were added to t-150 flasks and incubated overnight. The next day, the media was removed from the cells and replaced with fresh media (25mL). The cells were transiently co-transfected with pTGFβ-301LUC (10μg), pCMVER (1μg), and pRSVβgal (1μg) using the ProFection Mammalian Transfection System (Promega). A transfection solution was prepared by mixing pTGFβ-301LUC (940μg), pCMVER (94μg), pRSVβgal (94μg), $CaCl_2$ (5.704mL), and nuclease free water (47ml) with 2XHEPES (47ml) while vortexing. The resulting solution was incubated at room temperature for 30 minutes. This transfection solution (3mL) was added to each flask.

After incubating the cells with the transfection solution overnight, the media was removed and each flask washed with Ca/Mg free phosphate buffered saline solution (10mL, GIBCO). The washed cells were trypsinized (3mL trypsin/EDTA) to detach the cells. The detached cells were treated with fresh media (3mL). A pellet of the cells was prepared by centrifuging and removing the media and trypsin. The cells were resuspended in approximately 20mL of media and counted. The cells were diluted to a concentration of 500,000 cells/mL. Next, 50,000 cells (100μL) were added to each well of a 96-well plate, and the cells incubated overnight.

The test compounds were dissolved in dimethyl sulfoxide and diluted initially to 1:1000. This dilution was accomplished by adding 2μL of drug solution to 1000μL of media in a deep well microtiter plate (1:500 dilution), then 100μL of the dilute drug solution is added to 100μL of media already in the plate (1:2 dilution). For compound screening, the compounds are diluted 1:1000, as previously described, then further diluted 1:100. Alternatively, the minimum effective concentration is determined using an eight-dilution dose-response curve. The compounds and cells are incubated in 96-well plates overnight.

The media was removed from the cells and each well washed with Ca/Mg free phosphate buffered saline (200μL). The saline solution was removed and the cells lysed with 60μL of lysis buffer (100mM $KPO_4$, 0.2% Triton X-100, 1mM DTT, pH 7.8). The resulting solution was used to assay for luciferase or β-gal activity. The luciferase assay was performed substantially as described in Example VI, except the luciferin solution comprises 100mg luciferin, 9mL 1M Glycyl-gylcine, 36mL 40mM EGTA, 720mL 1M DTT, 5.4mL 1M $MgSO_4$, and 310mL $H_2O$. The results of these experiments are shown in tabular form in Table XII.

TABLE XII

| compound | TGFβ assay[a] | bone density[b] |
|---|---|---|
| raloxifene | + | + |
| 088074 | - | - |
| 156678 | + | + |
| 171147 | + | + |
| 309503 | - | - |

[a] "+" indicates the test compound produced a two-fold induction in normalized luciferase activity with an $ED_{50} < 10nM$

[b] "+" indicates the bone mineral density is statistically higher than ovariectomized animals

The above compounds were also tested for their ability to preserve bone mineral density in ovariectomized rats. Seventy-five day old female Sprague Dawley rats (weight range of 225 to 275 g) were obtained from Charles River Laboratories (Portage, MI). They were housed in groups of three and had *ad libitum* access to food (calcium context approximately 1%) and water. Room temperature was maintained at 22.2°C ± 1.7°C with a minimum relative humidity of 40%. The photoperiod in the room was 12 hours light and 12 hours dark.

One week after arrival, the rats underwent bilateral ovariectomy under anesthesia [44 mg/kg Ketamine and 5 mg/kg Xylazine (Butler, Indianapolis, IN) administered intramuscularly]. Treatment with vehicle, or a test compound was initiated on the day of surgery following recovery from anesthesia. Oral dosage was by gavage in 0.5 mL of 1% carboxymethylcellulose (CMC). Body weight was determined at the time of surgery and weekly thereafter and the dosage was adjusted with changes in body weight. Vehicle or treated ovariectomized (ovex) rats and non-ovariectomized (intact) rats were evaluated in parallel with each experimental group to serve as negative and positive controls.

The rats were treated daily for 35 days (6 rats per treatment group) and sacrificed by decapitation on the 36th day. The 35 day time period was sufficient to allow maximal reduction in bone density, measured as described herein. The right femurs were excised and scanned at the distal metaphysis 1 mm from the patellar groove with single photon absorptiometry. Results of the densitometer measurements represent a calculation of bone density as a function of the bone mineral context and bone width. Generally, ovariectomy of the rats caused a reduction in femur density of about 25% as compared to intact vehicle treated controls. The results of these experiments are shown in Table XII.

The results of these experiments show that the compounds that potently induce transcription from the raloxifene responsive element of promoter region of the TBFβ-3 gene, such as raloxifene, 156678, and 171147, also show preservation of bone density in ovariectomized rats.

The compounds that fail to induce transcription for the raloxifene responsive element, such as 088074 and 309503, also fail to show a statistically significant protection against bone loss in mineral density over ovariectomized rats.

It should be understood that the foregoing disclosure emphasizes certain specific embodiments of the invention and that all modifications or alternatives equivalent thereto are within the spirit or scope of the invention as set forth in the appended claims.

## Claims

1. A nucleic acid comprising a raloxifene responsive element isolated from a promoter region of a TGFβ gene wherein the human TGFβ gene is TGFβ-2 or TGFβ-3.

2. A nucleic acid according to Claim 1 wherein the nucleotide sequence of the nucleic acid comprises a sequence selected from the group consisting of sequences corresponding to 1896 to 2306 shown in Figure 3 and SEQ ID NO:3, sequences corresponding to 1976 to 2306 shown in Figure 3 and SEQ ID NO:3, sequences corresponding to 2106 to 2306 shown in Figure 3 and SEQ ID NO:3, sequences corresponding to 2137 to 2306 shown in Figure 3 and SEQ ID NO:3, sequences corresponding to 2150 to 2306 shown in Figure 3 and SEQ ID NO:3, sequences corresponding to 2159 to 2306 shown in Figure 3 and SEQ ID NO:3, sequences corresponding to 2159 to 2271 shown in Figure 3 and SEQ ID NO:3, and sequences

corresponding to 2159 to 2231 shown in Figure 3 and SEQ ID NO:3.

**3.** A recombinant expression construct comprising the nucleic acid according to Claim 1 or Claim 2 and a reporter gene.

**4.** A eukaryotic cell transfected with the recombinant expression construct according to Claim 3.

**5.** A method for screening a multiplicity of compounds to identify compounds having potential as anti-osteoporosis agents, the method comprising identifying a compound of the multiplicity that is capable of inducing transcription from a raloxifene responsive element of a mammalian promoter, that is not a non-specific transcription inducer, is not capable of inducing transcription from an estrogen-responsive element of a mammalian promoter and that is an anti-estrogenic or non-estrogenic compound, the method comprising the steps of:

(a) assaying for the ability of the compound to induce transcription from a raloxifene responsive element of the mammalian promoter;
(b) assaying for the inability of the compound to induce transcription from a mammalian promoter not having a raloxifene responsive element;
(c) assaying for the inability of the compound to induce transcription from an estrogen responsive promoter; and
(d) assaying for the ability of the compound to inhibit estrogen induction of transcription from an estrogen responsive promoter in the presence of estrogen.

**6.** A method for inducing bone formation in a mammal which comprises administering a compound that, when bound to an estrogen receptor, potently induces transcription from a raloxifene responsive element of a promoter region of a TGFβ-3 gene, provided the compound is other than a compound of the formula

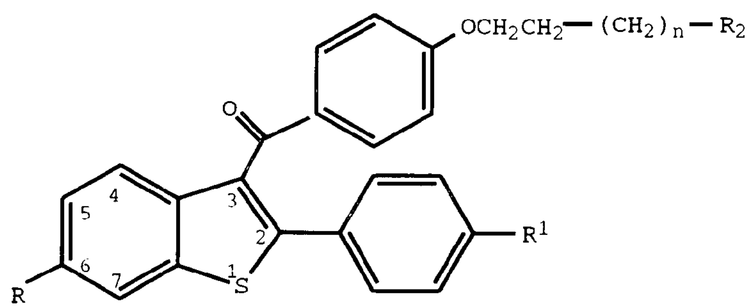

(I)

wherein

n is 0, 1 or 2;

R and $R^1$, independently, are hydrogen, hydroxyl, $C_1$-$C_6$-alkoxy, $C_1$-$C_6$-acyloxy, $C_1$-$C_6$-alkoxy-$C_2$-$C_6$-acyloxy, $R^3$-substituted aryloxy, $R^3$-substituted aroyloxy, $R^4$-substituted carbonyloxy, chloro, or bromo;

$R^2$ is a heterocyclic ring selected from the group consisting of pyrrolidino, piperidino, or hexamethyleneimino;

$R^3$ is $C_1$-$C_3$-alkyl, $C_1$-$C_3$-alkoxy, hydrogen, or halo; and

$R^4$ is $C_1$-$C_6$-alkoxy or aryloxy; or

a pharmaceutically acceptable salt thereof.

**7.** A method for treating osteoporosis which comprises administering a compound that, when bound to an estrogen receptor, potently induces transcription from a raloxifene responsive element of a promoter region of a TGFβ-3 gene, provided the compound is other than a compound of the formula

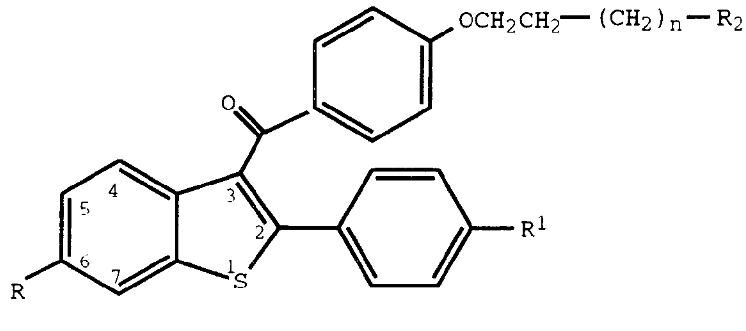

(I)

wherein

n is 0, 1 or 2;

R and $R^1$, independently, are hydrogen, hydroxyl, $C_1$-$C_6$-alkoxy, $C_1$-$C_6$-acyloxy, $C_1$-$C_6$-alkoxy-$C_2$-$C_6$-acyloxy, $R^3$-substituted aryloxy, $R^3$-substituted aroyloxy, $R^4$-substituted carbonyloxy, chloro, or bromo;

$R^2$ is a heterocyclic ring selected from the group consisting of pyrrolidino, piperidino, or hexamethyleneimino;

$R^3$ is $C_1$-$C_3$-alkyl, $C_1$-$C_3$-alkoxy, hydrogen, or halo; and

$R^4$ is $C_1$-$C_6$-alkoxy or aryloxy; or

a pharmaceutically acceptable salt thereof.

8. A method for treating bone fractures which comprises administering a compound that, when bound to an estrogen receptor, potently induces transcription from a raloxifene responsive element of a promoter region of a TGFβ-3 gene, provided the compound is other than a compound of the formula

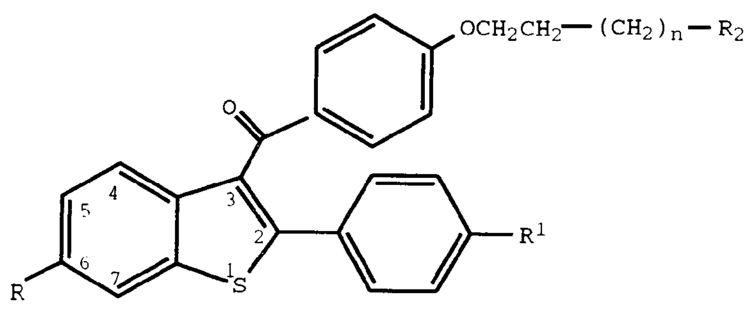

(I)

wherein

n is 0, 1 or 2;

R and $R^1$, independently, are hydrogen, hydroxyl, $C_1$-$C_6$-alkoxy, $C_1$-$C_6$-acyloxy, $C_1$-$C_6$-alkoxy-$C_2$-$C_6$-acyloxy, $R^3$-substituted aryloxy, $R^3$-substituted aroyloxy, $R^4$-substituted carbonyloxy, chloro, or bromo;

$R^2$ is a heterocyclic ring selected from the group consisting of pyrrolidino, piperidino, or hexamethyleneimino;

$R^3$ is $C_1$-$C_3$-alkyl, $C_1$-$C_3$-alkoxy, hydrogen, or halo; and

$R^4$ is $C_1$-$C_6$-alkoxy or aryloxy; or

a pharmaceutically acceptable salt thereof.

9. A method for inducing bone formation in a mammal which comprises administering a compound that:

(a) potently induces transcription from a raloxifene responsive element of a promoter region of a TGFβ-3 gene;

(b) does not induce transcription from a mammalian promoter not having a raloxifene responsive element;

(c) does not induce transcription from an estrogen responsive promoter; and
(d) inhibits estrogen-induced transcription from an estrogen responsive promoter in the presence of estrogen;
provided the compound is other than a compound of the formula

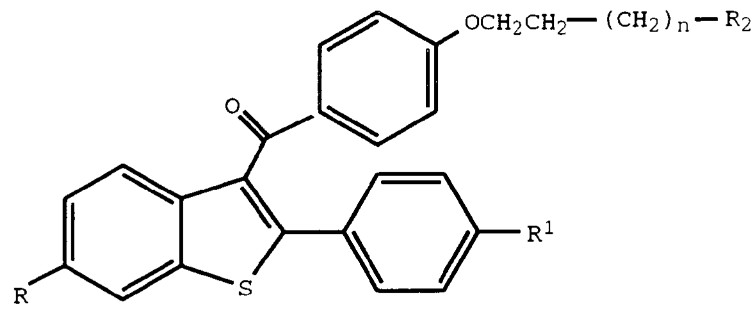

(I)

wherein
n is 0, 1 or 2;
R and $R^1$, independently, are hydrogen, hydroxyl, $C_1$-$C_6$-alkoxy, $C_1$-$C_6$-acyloxy, $C_1$-$C_6$-alkoxy-$C_2$-$C_6$-acyloxy, $R^3$-substituted aryloxy, $R^3$-substituted aroyloxy, $R^4$-substituted carbonyloxy, chloro, or bromo;
$R^2$ is a heterocyclic ring selected from the group consisting of pyrrolidino, piperidino, or hexamethyleneimino;
$R^3$ is $C_1$-$C_3$-alkyl, $C_1$-$C_3$-alkoxy, hydrogen, or halo; and
$R^4$ is $C_1$-$C_6$-alkoxy or aryloxy; or
a pharmaceutically acceptable salt thereof.

**10.** The use of a compound that, when bound to an estrogen receptor, potently induces transcription from a raloxifene responsive element of a promoter region of a TGFβ-3 gene, provided the compound is other than a compound of the formula

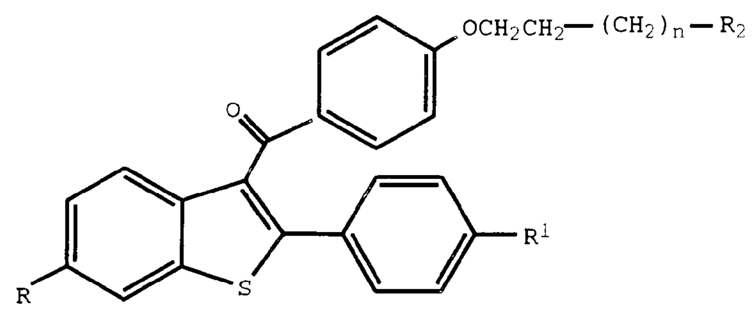

(I)

wherein
n is 0, 1 or 2;
R and $R^1$, independently, are hydrogen, hydroxyl, $C_1$-$C_6$-alkoxy, $C_1$-$C_6$-acyloxy, $C_1$-$C_6$-alkoxy-$C_2$-$C_6$-acyloxy, $R^3$-substituted aryloxy, $R^3$-substituted aroyloxy, $R^4$-substituted carbonyloxy, chloro, or bromo;
$R^2$ is a heterocyclic ring selected from the group consisting of pyrrolidino, piperidino, or hexamethyleneimino;
$R^3$ is $C_1$-$C_3$-alkyl, $C_1$-$C_3$-alkoxy, hydrogen, or halo; and
$R^4$ is $C_1$-$C_6$-alkoxy or aryloxy; or
a pharmaceutically acceptable salt thereof; in the preparation of a medicament useful for treating osteoporosis.

# FIG. 1A

```
-1362 GGATCCTTAGCAGGGGAGTAACATGGATTTGGAAAGATCACTTTGGCTGCTGTGTGGGGATAGATAAGACGGTGGGAGCCTAGAAAGGAGGCTGGGTTGG

-1262 AAACTCTGGGACAGAAACCCAGAGAGGAAAAGACTGGGCCTGGGGTCTCCAGTGAGTATCAGGGAGTGGGGAATCAGCAGGAGTCTGGTCCCCACCCATC

-1162 CCTCCTTTCCCCTCTCTCTCCTTTCCTGCAGGCTGGCCCCGGCTCCATTTCCAGGTGTGGTCCCAGGACAGCTTTGGCCGCTGCCAGCTTGCAGGCTATG

-1062 GATTTTGCCATGTGCCCAGTAGCCCGGGCACCCACCAGCTGGCCTGCCCCACGTGGCGGCCCCTGGGCAGTTGGCGAGAACAGTTGGCACGGGCTTTCGT

 -962 GGGTGGTGGGCCGCAGCTGCTGCATGGGGACACCATCTACAGTGGGGCCGACCGCTATCGCCTGCACACAGCTGCTGGTGGCACCGTGCACCTGGAGATC

 -862 GGCCTGCTGCTCCGCAACTTCGACCGCTACGGCGTGGAGTGCTGAGGGACTCTGCCTCCAACGTCACCACCATCCACACCCCGGACACCCAGTGATGGGG

 -762 GAGGATGGCACAGTGGTCAAGAGCACAGACTCTAGAGACTGTCAGAGCTGACCCCAGCTAAGGCATGGCACCGCTTCTGTCCTTTCTAGGACCTCGGGGT

 -662 CCCTCTGGGCCCAGTTTCCCTATCTGTAAATTGGGGACAGTAAATGTATGGGGTCGCAGGGTGTTGAGTGACAGGAGGCTGCTTAGCCACATGGGAGGTG

 -562 CTCAGTAAAGGAGAGCAATTCTTACAGGTGTCTGCCTCCTGACCCTTCCATCCCTCAGGTGTCCTGTTGCCCCCTCCTCCCACTGACACCCTCCGGAGGC
```

# FIG. IB

-462 CCCCATGTTGACAGACCCTCCTTCTCCTACCTTGTTTCCCAGCCTGACTCTCCTTCCGTTCTGGGTCCCCCTCCTCTGGTCGGCTCCCCTGTGTCTCATC

-362 CCCCGGATTAAGCCTTCTCCGCCTGGTCCTCTTTCTCTGGTGACCCACACCGCCCGCAAAGCCACAGCGCATCTGGATCACCCGCTTTGGTGGCGCTTGG

-262 CCGCCAGGAGGCAGCACCCTGTTTGCGGGGCGGAGCCGGGGAGCCCGCCCCCTTTCCCCCAGGGCTGAAGGACCCCCCTCGGAGCCCGCCCACGCGAGA

-162 TGAGGACGGTGGCCCAGCCCCCCCATGCCCTCCCCCTGGGGCCCGCCCCCGCTCCCGCCCCGTGCGCTTCCTGGGTGGGGCCGGGGGCGGCTTCAAAACC

+1

-1

-62 CCCTGCCGACCCAGCCGGTCCCCGCCGCCGCCGCCCTTCGCGCCCTGGCCATCTCCCTCCCACCTCCCTCCGCGGAGCAGCCAGACAGCGAGGGCCCCG

39 GCCGGGGGCAGGGGGACGCCCCGTCCGGGGCACCCCCCCGGCTCTGAGCCGCCCGCGGGCCGGCCTCGGCCCGGAGCGGAGGAAGGAGTCGCCGAGGA

139 GCAGCCTGAGGCCCCAGAGTCTGAGACGAGCCGCCGCCGCCCCCGCCACTGCGGGGAGGAGGGGGAGGAGGAGCGGGAGGAGGGACGAGCTGGTCGGGAG

+271

239 AAGAGGAAAAAAACTTTTGAGACTTTTCCGTTGCCGCTGGGAGCCGGAGGCGCGGGGACCTCTTGGCGCGACGCTGCCCCGCGAGGAGGCAGGACTTGGG

339 GACCCCAGACCGCCTCCCTTTGCCGCCGGGGACGCTTGCTCCCTCCCTGCCCCCTACACGGCGTCCCTCAGGCGCCCCCATTCCGGACCAGCCCTCGGGA

+470 +525

439 GTCGCCGACCCGGCCTCCCGCAAAGACTTTTCCCCAGACCTCGGGCGCACCCCCTGCACGCCGCCTTCATCCCCGGCCTGTCTCCTGAGCCCCCGCGCAT

539 CCTAGACCCTTTCTCCTCCAGGAGACGGATCTCTCTCCGACCTGCCACAGATCCCCTATTCAAGACCACCCACCTTCTGGTACCAGATCGCGCCCATCTA

FIG. IC

```
639 GGTTATTTCCGTGGGATACTGAGACACCCCCGGTCCAAGCCTCCCCTCCACCACTGCGCCCTTCTCCCTGAGGAGCCTCAGCTTTCCCTCGAGGCCCTCC

739 TACCTTTTGCCGGGAGACCCCCAGCCCCTGCAGGGGCGGGGCCTCCCCACCACACCAGCCCTGTTCGCGCTCTCGGCAGTGCCGGGGGGCGCCGCCTCCC

       Met
839 CCATG
```

FIG. 2A

-2277 AAGCTTTTACCAATACCTCCCGTCTTACCCCTCCTGGGCTTTGGGGAAATTAAAGTAGCCTCTTATGAGTAAGTCAG

-2200 GGGTCTCAGGTCTAAGGAGTTGTTAAGTGAGCAATAGGTACTCAGTAAACTAAGTATTATGAACAAAAGTGTGTATGTCTATGTCAGGAAGAGGGGTGGC

-2100 CATCAGAATTTATGGCTTGCGCTGTTTCCTAGAAGTGATGTAATGAACTTTTGCTACTCTATCCACACTCTAATCTGAATCTACTTAAGGTGCATCAGTG

-2000 TCTGTACCAAGAAGGTTGTCTATAAACATGAAAGATGGATGCTCACTGGCTTGTGGAAGCTGAACCTGTATCCTCAGAAAATACAGTGATAGCTAATTCA

-1900 GGTAACCAGCCATATTCCACAGCAGCATCTTCTCTCAGTAGCTCTGGTTTGGAGCTCCTGCTCTGTGTCTATAATGGCCACAGGTGTAAGAATATTCACT

-1800 TTTTGTCCAATCTGTAGAGCTAGCCTACTGCAGTTCTCAAACTGAACTCAGAGGGAGGACCTAACTGGATGAAACTACTAGTCTGACAGTAGCGCCTCTT

-1700 GATTATCTTTTTCTTGGGCTACTGGGATGGTAGCTTTGCTTCAACTCAAAACTGGTATCAAGGAAAGGAACCTGCTGGTGCTGATTTATACATAATTTTT

-1600 AGAATTATTCAGAAGTGGGTTGGAACAATTATTTATTCCAGAGTTTTTCAATGTGTGATAATGGAAAAAATTCTGTATTCAAGGGAGTTTGGAAAATGC

-1500 TGGGTTAAAAGAGTGAAAAGGTTTTCTCTTCTACAGGAGTTTCAGAGCCTTTAACATGATAATGTTCCAGAATGAGGAATCTAAGAGGACAGGAGAGTAC

-1400 CCAGTATCTCCCAAACTTGTTTGACTCCAGAATTCCTGTTTGTCAGAACATATTCTGGGACCATTGTTTCTCAGAAGTACATAGTAGGTAAGAACATAGT

# FIG. 2B

-1300 GGATCCTGACTGCAAAAATCCAGCTCTACCACTTACTGTGGTCTCGAACAAAGTACTTAACTTCTTTGTACCTCAGTCTCCTCATCTGCCAGATATGGAT

-1200 AATAAGACCCACTTTATAGGTTCATAGTGAAGATTAAATGACCATACACAACACACATCAAATTACTAAGTGTAGTATATGTTAGCTATTATTATTTTAT

-1100 TTATTCAGTGCTCTACTAATAACCTAGGCCCCATACACAACTGAAGTATAATTCCAAAAGTGATAGAAAGTTCTTTGTGACTTTTCTGAACTCAGGAACA

-1000 TCTGAAGTAGAGAACAGTATAGAGATCTTGGGTTTGGGAGTACATTCAACAGAGTTTTCCAGTTTAAATCATCTGTCTGGTCAGTATGGCTGCAGAGTCA

-900 TGCCGAAATGAAAATGTTGACTTTGAGTAACTAAAGGTAAAATAAAAGAAAAAGGGAAGGTGGAACAGTGGTAAGAGTTATTCTGTATTCATCTAATTTA

-800 AGACTTAGTTGAAATTGAAAATGTCAAGTTATGAGTAGTGTAGAACAAGTAGACATCAAACACTTAAAATTCCAGCTTCCTGGATTATGCTATGGAAAGA

-700 ATGAAGTTGGTGGATAATGTTTAGCCTAGCAAGAAGGTCAAGAAGAAGAAAGCCATACAAGAAGTGGCTTAGGCAGCAAATTATAAAGGTGACCATTCAT

-600 TCAAATCAGTAAAACAAACAAGTATACCTTATTCTTTAGGTAAAATTGATGGATCTCTGTTTTCCAGCAGTTCACAAACAGAGGGGTACATTGTAAACAA

-500 CAAACTAACAAAATAAATTCTGGGATGGCAACCTGCTAAGGTATCCCAGAAAATAAGAGGTAGGACATGAATTTAAAAGATTGGAAGGTATGTCTTCAGT

-400 ACTGGCCTGGCCCTGAGTAGACTAGTGCCCTCCATAGGGGTGCGTGTGCACACATAATACAGGAGGGAAGCCTTCCCTTCTAGAGCAAGTGATTCAGCTT

# FIG. 2C

-300 GGGAGGCTGTGACTGAGCTACACTAAGTAAAAACGGGAGACTTGATTGTCCTTCAACAGACCTGTCCAAAATGACTGGAAAGTAAATACCGTAAATCACT

-200 GTTGTCAGGGCGCACATTCCACCTCCTTCCTCCCTTACCCACAGCGGTCCACATTTCCACACTCCCTACACGGTTCGGGGAGAGCTCGTGGTCTAAGTAA

-100 CGAGAGGACTTCTGACTGTAATCCTAGCACGTCACTTTGTTGAAGGCAGACACGTGGTTCAGAGAGAACTTATAAATCTCCCCTCCCCGCGAAGATCGTG

-1 ATGTTATTCGCTGGCAGCAGAAGGTCTTGCCGAGCGAGCTCCAGAACGTCCTGACAAGAGAAAGACAGATTGAGATAGAGATAGAAAGAGAAAGAGAGAA

101 AGAGACAGCAGAGCGAGAGCGCAAGTGAAAGAGGCAGGGGAGGAGGGGATGGAGCATATTACGTGACCGGCCTAGGGAGTCATCCAGGAACAAACTGAG

201 GGGCTGCCCGGCTGCAGACAGGAGGAGACAGAGAGGATCTATTTTAGGGTGGCAAGTGCCTCCTACCCTAAGCGAGCAATTCCACGTTGGGGAGAAGCCA

301 GCAGAGGTTGGGAAAGGGTGGAGTCCAAGGGACGCCCCTGCGCAACTCCCTCAGGAATAAAACTCCCCAGCCAGGGTGTCGCAAGGGCTGCCGTTGTGA

401 TCCGCAGGGGGTGAACGCAACCGCGACGGCTGATCGTATGTGGCTGGGTTGGCGTTTGGAGCAAGAGAAGGAGGAGCAGGAGAAGGAGGGAGCTGGAGGC

# FIG. 2D

501 TGGAAGCGTTTGCAAGCGGCGGCGGCAGCAACGTGGAGTAACCAAGCGGGTCAGCGCGCGCGCCAGGGTGTAGGCCACGGCGCGCAGCTCCCAGAGCA

601 GGATCCGCCCGCCCTCCGCAGCCTCTGCGGCCCCTGCGGCACCCGACCGAGTACCGAGCGCCCTGCGAACGGCACCCTCCTCCCCGCGGTGGCTGGGCTC

701 GCCCCAGCGCGCACACGCACACACACACACACACACACACGCACGCACACACGTGTCGTTCTCTGCTCCGGAGCTGCTGCTGCTCCTGCTCTCAGCGC

801 CGCAGTGGAAGGCAGGACCGAACCGCTCCTTCTTTAAATATATAAATTTCAGCCCAGGTCAGCCTCGGCGGCCCCCCTCACCGCGCTCCCGCCCCTCCCG

901 TCAGTTCGCCAGCTGCCAGCCCCGGGACCTTTTCATCTCTTCCCTTTTGGCCGGAGGAGCCGAGTTCAGATCCGCCACTCCGCACCCGAGACTGACACAC

1001 TGAACTCCACTTCCTCCTCTTAAATTTATTTCTACTTAATAGCCACTCGTCTCTTTTTTTCCCCATCTCATTGCTCCAAGAATTTTTTTCTTCTTACTCG

1101 CCAAAGTCAGGGTTCCCTCTGCCCGTCCCCTATTAATATTTCCACTTTTGGAACTACTGGCCTTTTCTTTTTAAAGGAATTCAAGCAGGATACGTTTTTC

1201 TGTTGGGCATTGACTAGATTGTTTGCAAAAGTTTCGCATCAAAAACAACAACAACAAAAAACCAAACAACTCTCCTTGATCTATACTTTGAGAATTGTTG

Met His Tyr Cys Val Leu Ser Ala Phe Leu Ile
1301 ATTTCTTTTTTTATTCTGACTTTTAAAAACAACTTTTTTTTCCACTTTTTTAAAAAATG CAC TAC TGT GTG CTG AGC GCT TTT CTG ATC

# FIG. 3A

1 CAGTAGTAGC TTCCAGAACT TGCTTAGCAC CTGAATCACG TGTGAGGTTT

51 GTAAAGAAAC AGAGATGCCA GGGCCTCAGC TCTGGAGACT GATTGGTAGA

101 GGTGGAGTCC AAAAAAGTAT AACTTTAATA ATTTTCCTTC CTATCTTCAA

151 CTGTCTGCTC AAAGGCCTTC CCTTATCACC CTATTTGAAA CTGCAACATC

201 CCCCAACCTA GGCACACCCC ATCCTCCTTC CCTGCTTGAT TTTCTGCCAC

251 ACCACATTTG TTTGTTTGCT TGTCTGTTTG AGACACGGTC TTGCTCTGTC

301 GTCCAGGCTG GAGTGCAGTG GTGCAATCTT GGCCCCCTGT AAACTCGCCT

351 CCCTGGCTCA AGTGATTATC CTGCTCAGCC TCCCAAGTAG ATGCGTGCGC

401 CAACATGCCG GGCTAATTTT TCCATTTTTT TGTAGAGACT GGGTTTCGCC

451 GTGTTGCTGG GGCTGGTCTC GAATTCCTGA GCTCAAGTAA TCCTCCTGCA

501 TGGGCCTCCC CAAATGCTGG GATTACAGGC GTGAGCCACT GCACCTGGCT

551 CAGCACTTTT TACCGTACTA CATCATTTAC ATATTTATTT AGTTTATCGC

601 CTCCTCCACT GCCCCACCCC TGCCTCTAAA TAAAATTTCC CTGAGGGCAG

651 GAGTTTTGTT TCGTTCACTG ATATTCTTCA CAGAGCCTAG AATAGTGCCT

701 GGTATATAGA AACATTAAAC TTTTTCTGAA ATTTCAGAGG CAGTATAGCA

751 TAGTAATTAA GTCCAGAATC TGGCAACGTC CTGGGTGCAA ATCCCAACAG

801 CTGACACCTA ATAACTATGT GACCTTGGGC AAGTTACTTT TAAAGTTTCT

# FIG. 3B

851 ACCCCTAGGT TTCCCATTGG TTTTGCAAAT GAAAGTAATG CCTACCCAAG

901 CTAGATAGCC TGTGTAAATA TCGCCTCCAT CACTCACAAG CAGTGTGGTC

951 TGTAAAAAAA AAAACAAAAA ACTCTATGCC TCAGTTTCCT CATCCGTAAA

1001 AGTGACCCAC CGCTGTGCTG GGATACAGAG AACAGCCCCT TCAGTTAGTG

1051 GCCTGGAAGC CAGCCTCTCA GAAAGGGTCC AGGAAGGCTG GAGTGAGATG

1101 GGGTGGAGCG GCACTCACTC TCAGGAAAGT TCAGTTCAGA GGCAAGCCCT

1151 GTGTTGCGGG GTGCGGGGAG CCACGTGCCC TACCCTCCCT TGGCTGCTCG

1201 TGGGAAAAGG CCTAGAGGTT CGGGCCGAGA AGAGGAGCGA AAGCACAGAG

1251 CCGACTTCCC CTCACCCATC TGGGAAATGG CTCGGGCCAA CTGCTGACTT

1301 CGCGCTCGCT GGCCGACGTC CTGCGGAGAC CTCGGCGGGG AGGGAGGCTG

1351 AACATCTGGA TGACATTTCT GCGAGAGAGC GGCTCCGGAG CGGCGGTCGG

1401 GGAGGGAGAG CTGCTCGTGC GCACGTCGGG CCGGGAGGGA GGCGATTCCT

1451 CGGGGCCTGG GTCTTGTTTT TCTCGCTCTC TACCGCAGCC CCTTCTCCCG

1501 CCCCTCAGCC CCCACCCCGC AGCCCCCAGC CCCCGAGCCT CCCCGGCTCC

1551 CGACCAGCCG AGCTCCTTCA CTGGCGGCCT CCGCTCGCCA GAGGGCACCC

1601 TCGATCTTCC GGAAAACGCC ACCATTTTTC ACTGCCCCTG GAGCGTCTCC

1651 AGGCTTCTGC CCGCCTCCCG ACTCCGATCT TGTCAATGAA GAATCGGGCC

# FIG. 3C

1701 AGGATCGCCG CGGAGCGGAC GCCGACCCTC CGACCCGGCT CGCAGGCTGG

1751 GAGTCCCCTC TGCGAGGCTG GCATGGCCGC CCCTACCGGG TCCCGCGCCC

1801 TCTGCGGACC CTCCCCGGGT TGGGCCTGGC CGCGGGCGGC CCCGGGACCG

-301

1851 GGGGACCAGG AGGGAGAGTA GACCGGGCCG GACGGCGCGG ACTGACAGCT

1901 GGCGAGAGGG CGCCGGGGCT GGGGGAAAGG GAGGGAGGGG GCTCATCGGA

-221

1951 GTAACTTTCC AGAAAAACAC CAACGTGTGG CAGGAGTGAT TCCAAGAGGG

2001 GAAAAAAAGT TCAGCTACCA CGTCGAACGA GAGGACTCGC AAAGTATTTT

2051 TCAAAAGGGC TCGGCTTTTC CTGTGCCTGT TTAAAACATT AACATCGTGC

-91 -60 -47

2101 AGCAAAAGAG GCTGCGTGCG CTGGTCCCTC CCTCCCCCAC CCCAGGCCAG

-38 +1

2151 AGACGTCATG GGAGGGAGGT ATAAAATTTC AGCAGAGAGA AATAGAGAAA

+35

2201 GCAGTGTGTG TGCATGTGTG TGTGTGTGAG AGAGAGAGGG AGAGGAGCGA

+75

2251 GAGGGAGAGG GAGAGGGAGA GAGAGAAAGG GAGGGAAGCA GAGAGTCAAG

+110

2301 TCCAAGGGAA TGACCGAGAG AGGCAGAGAC AGGGGAAGAG GCGTGCGAGA

2351 GAAGGAATAA CAGCAGCTTT CCGGAGCAGG CGTGCCGTGA ACTGGCTTCT

2401 ATTTTATTTT ATTTTTTTCT CCTTTTTATT TTTTAAAGAG AAGCAGGGGA

2451 CAGAAGCAAT GGCCGAGGCA GAAGACAAGC CGAGGTGCTG GTGACCCTGG

2501 GCGTCTGAGT GGATGATTGG GGCTGCTGCG CTCAGAGGCC TGCCTCCCTG

# FIG. 3D

2551 CCTTCCAATG CATATAACCC CACACCCCAG CCAATGAAGA CGAGAGGCAG

2601 CTGAAAAAGT CATTTAGAAA GCCCCCGAGG AAGTGTAAAC AAAAGAGAAA

2651 GCATGAATGG AGTGCCTGAG AGACAAGTGT GTCCTGTACT GCCCCACCTT

2701 TAGCTGGGCC AGCAACTGCC CGGCCCGCTT CTCCCCACCT ACTCACTGGT

2771 GATCTTTTTT TTTTTACTTT TTTTTCCCTT TTCTTTTCCA TTCTCTTTTC

2801 TTATTTTCTT TCAAGGCAAG GCAAGGATTT TGATTTTGGG ACCCAGCCAT

2851 GGTCCTTCTG CTTCTTCTTT AAAATACCCA CTTTCTCCCC ATCGCCAAGC

2901 GGCGTTTGGC AATATCAGAT ATCCACTCTA TTTATTTTTA CCTAAGGAAA

2951 AACTCCAGCT CCCTTCCCAC TCCCAGCTGC CTTGCCACCC CTCCCAGCCC

3001 TCTGCTTGCC CTCCACCTGG CCTGCTGGGA GTCAGAGCCC AGCAAAACCT

3051 GTTTAGACAC ATGGACAAGA ATCCCAGCGC TACAAGGCAC ACAGTCCGCT

3101 TCTTCGTCCT CAGGGTTGCC AGCGCTTCCT GGAAGTCCTG AAGCTCTCGC

3151 AGTGCAGTGA GTTCATGCAC CTTCTTGCCA AGCCTCAGTC TTTGGGATCT

3201 GGGGAGGCCG CCTGGTTTTC CTCCCTCCTT CTGCACGTCT GCTGGGGTCT

3251 CTTCCTCTCC AGGCCTTGCC GTCCCCCTGG CCTCTCTTCC CAGCTCACAC

3301 ATG

FIG.4

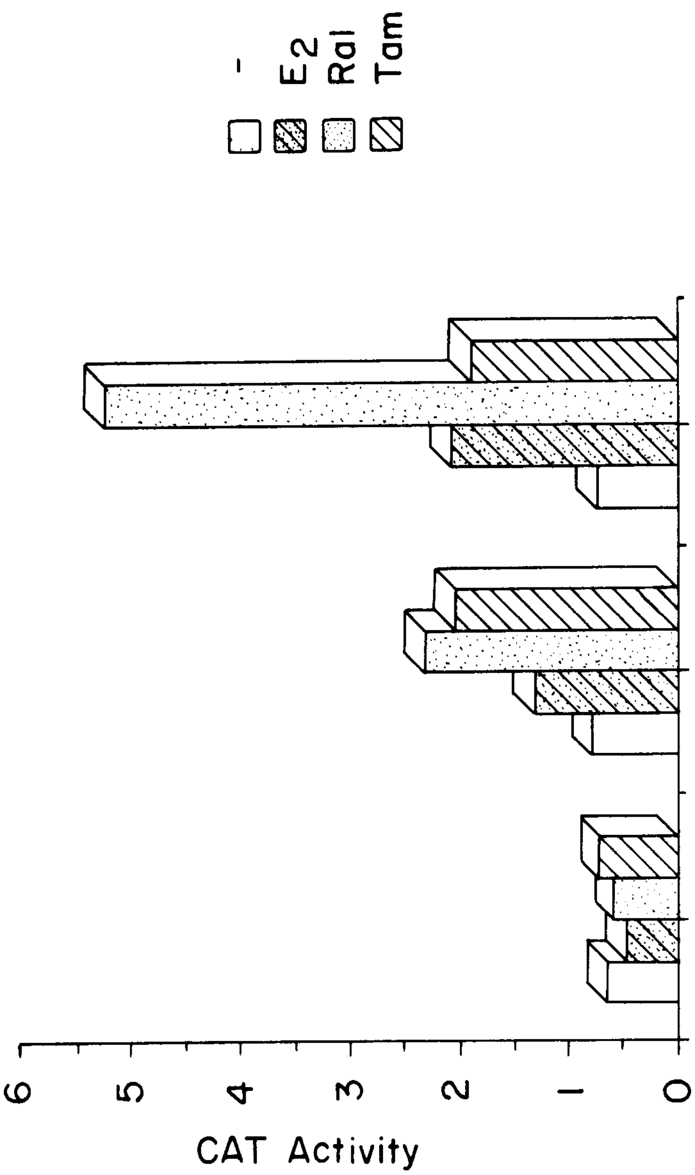

# FIG. 5A

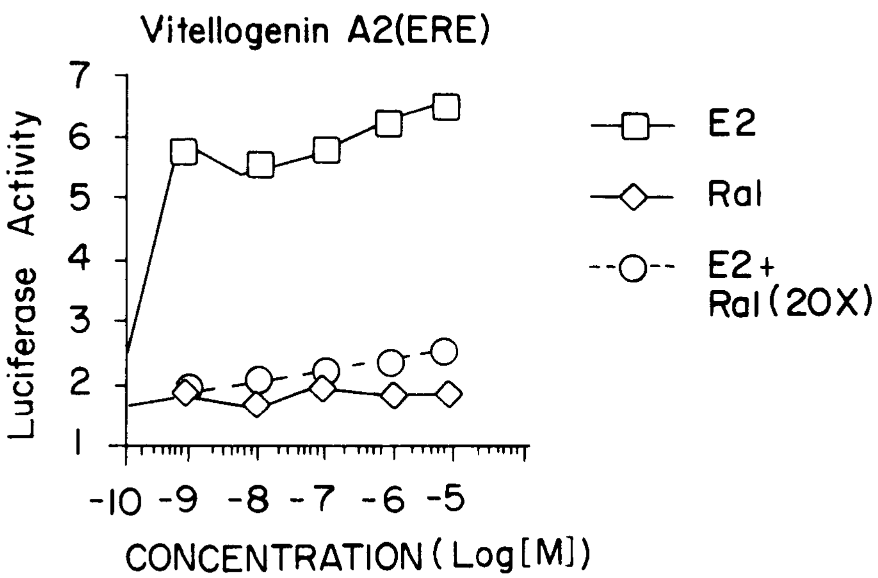
Vitellogenin A2(ERE)
Luciferase Activity
7
6
5
4
3
2
1
-10 -9 -8 -7 -6 -5
CONCENTRATION (Log[M])
E2
Ral
E2 +
Ral (20X)

# FIG. 5B

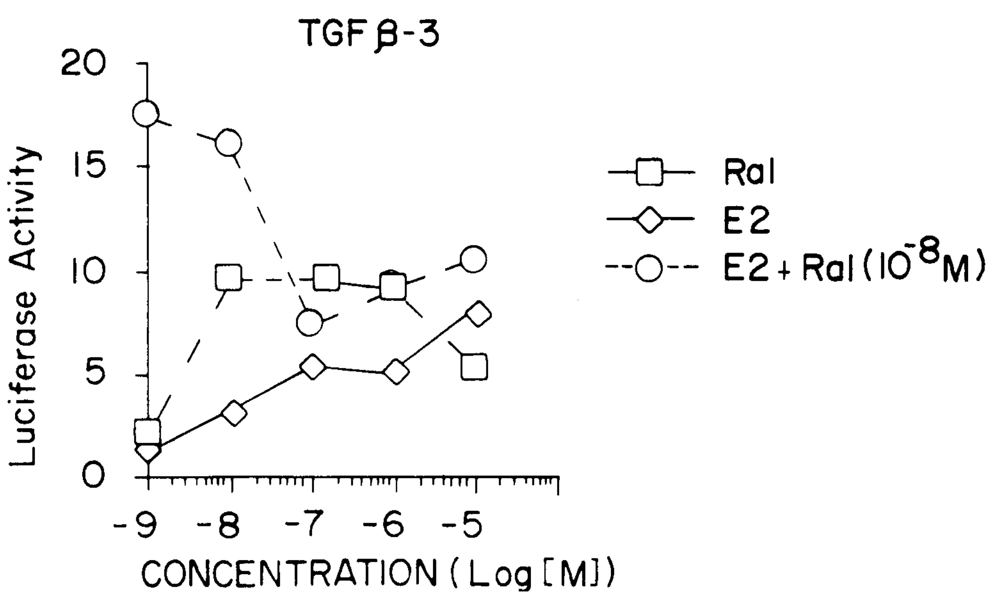
TGF β-3
Luciferase Activity
20
15
10
5
0
-9 -8 -7 -6 -5
CONCENTRATION (Log [M])
Ral
E2
E2 + Ral (10-8M)

FIG.6

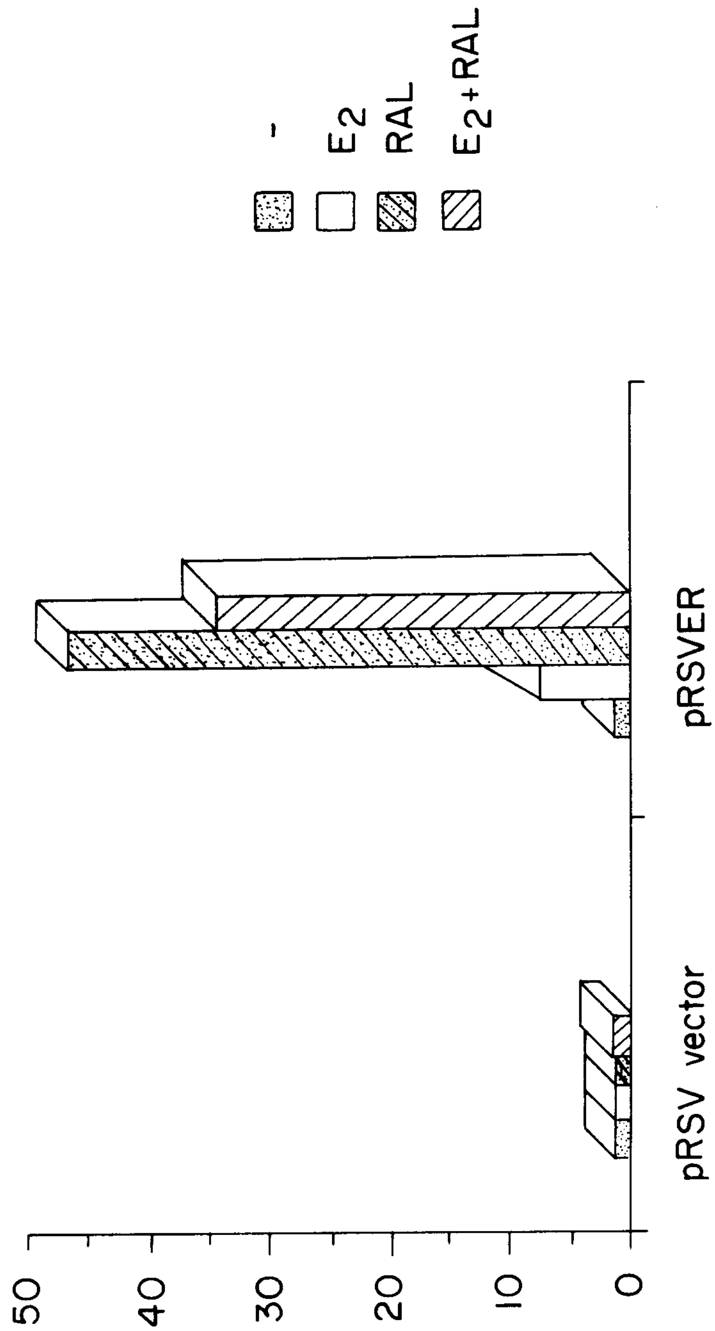

FIG. 7

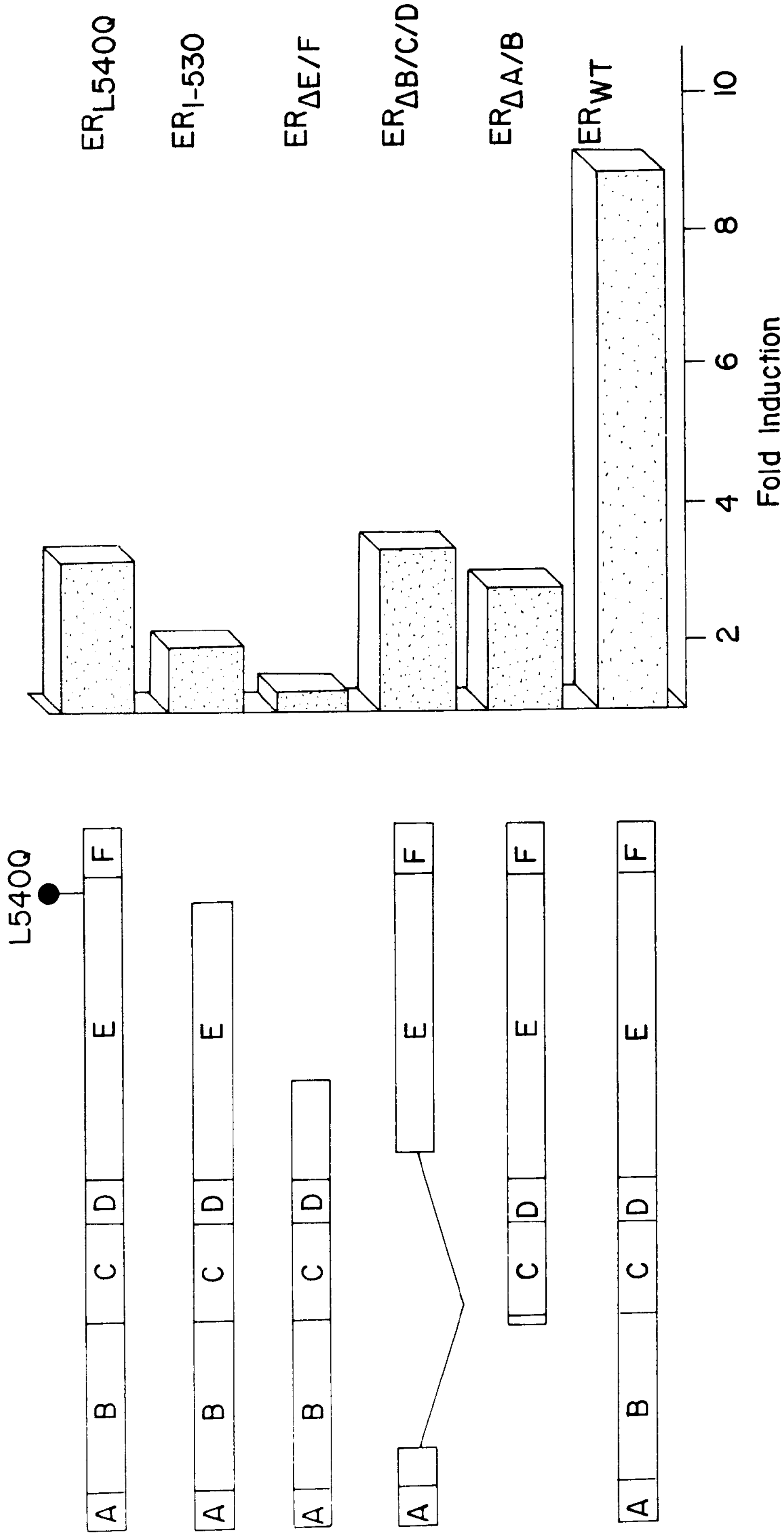
L540Q
A
B
C
D
E
F
ER L540Q
ER 1-530
ER ΔE/F
ER ΔB/C/D
ER ΔA/B
ER WT
2
4
6
8
10
Fold Induction

FIG.8

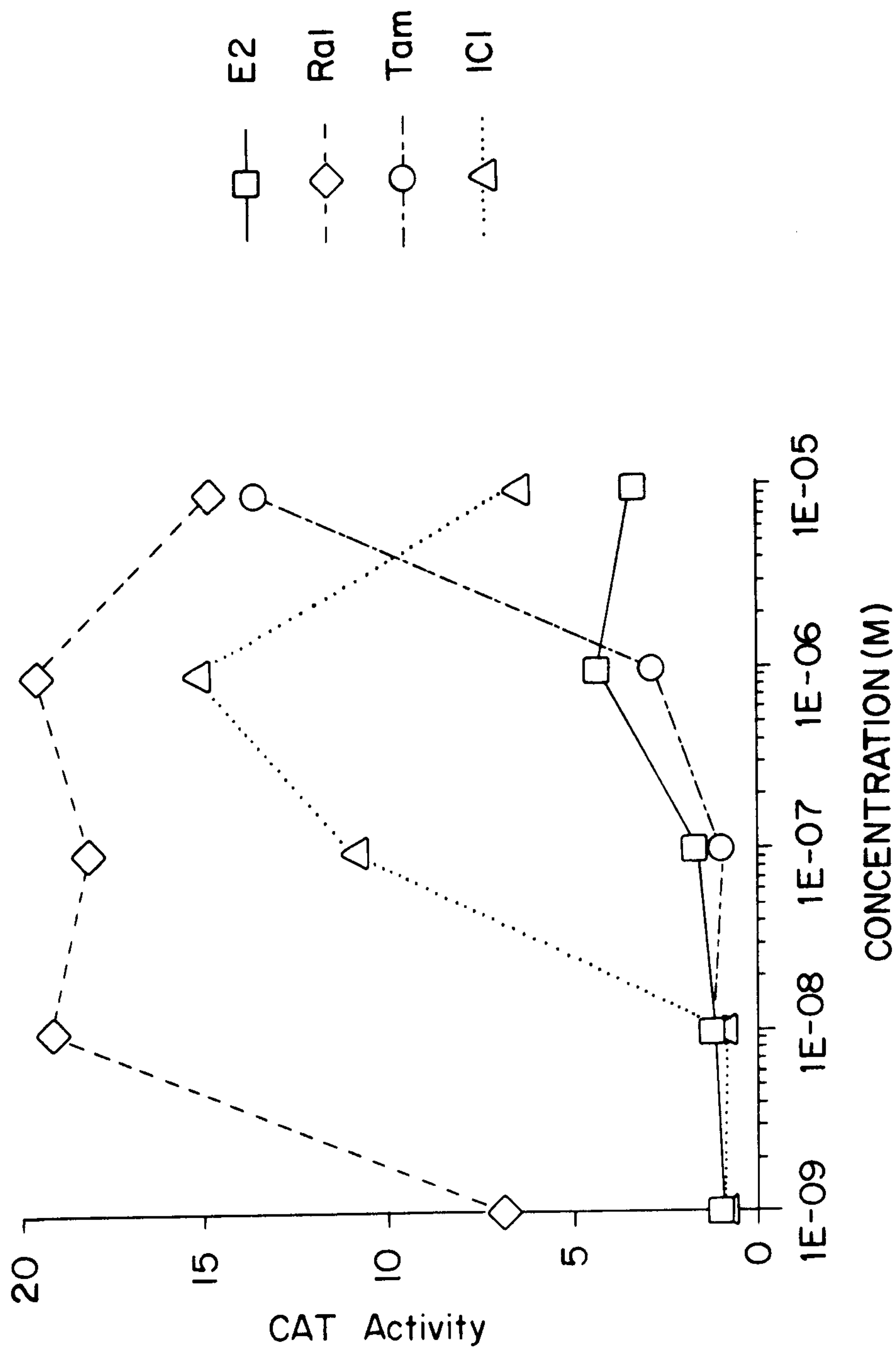
E2
Ral
Tam
ICI
CAT Activity
20
15
10
5
0
1E-09
1E-08
1E-07
1E-06
1E-05
CONCENTRATION (M)

FIG. 9

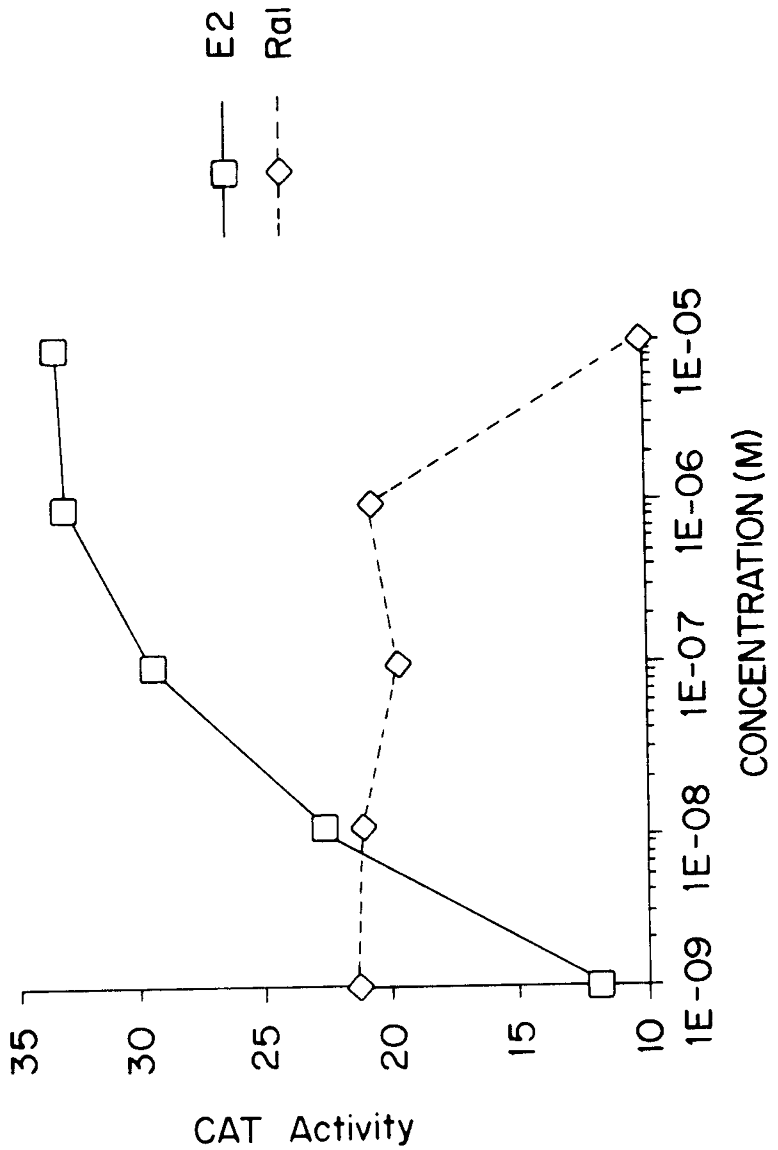

CAT Activity
35
30
25
20
15
10
1E-09
1E-08
1E-07
1E-06
1E-05
CONCENTRATION (M)
E2
Ral

FIG.10

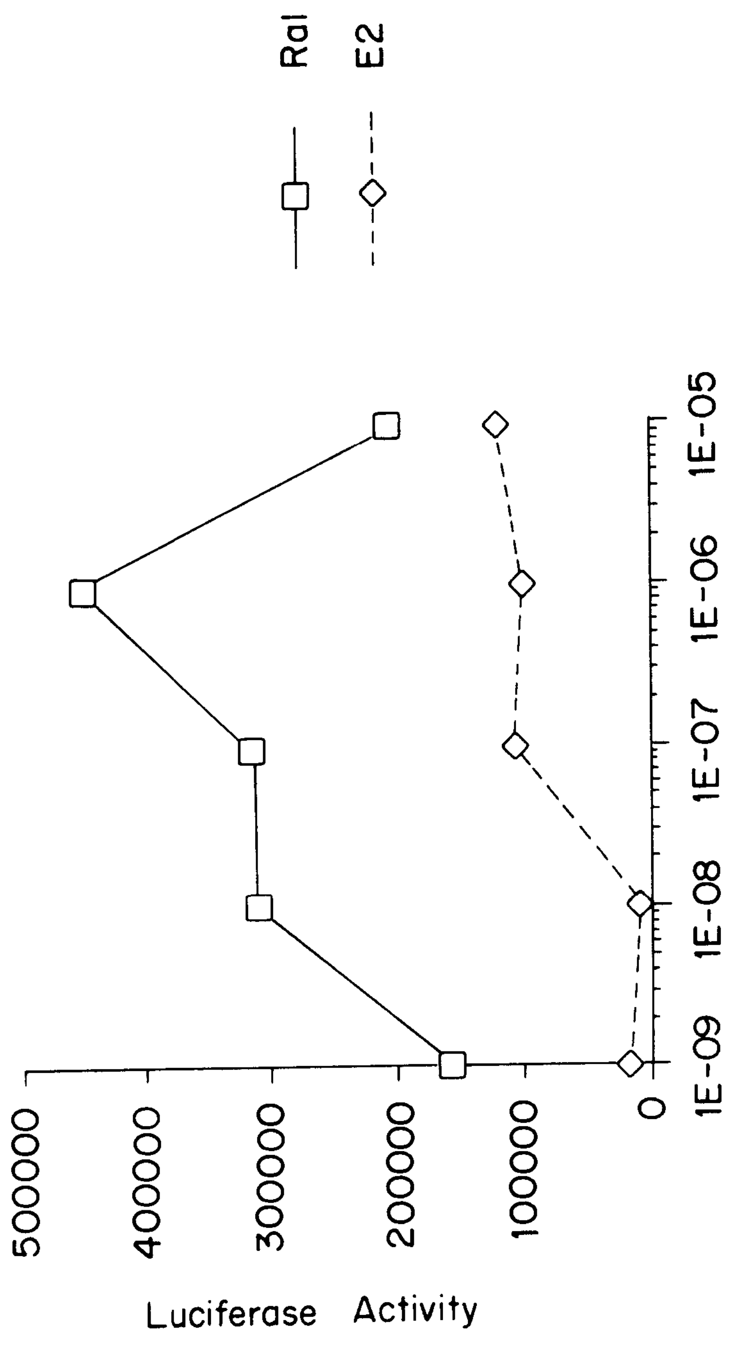
Luciferase Activity
500000
400000
300000
200000
100000
0
1E-09
1E-08
1E-07
1E-06
1E-05
CONCENTRATION (M)
Ral
E2

# FIG. 11

Estrogenic

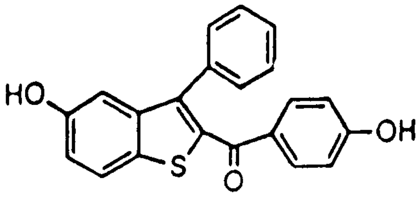

112676

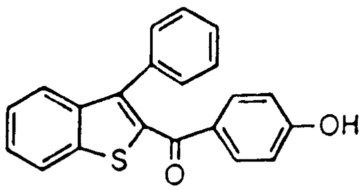

81099

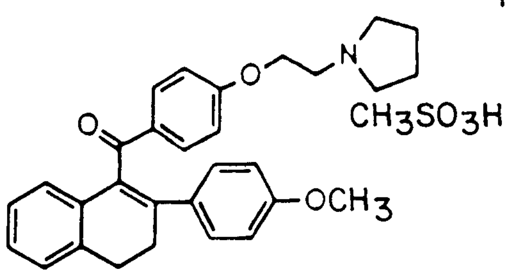

133314

Non-estrogenic

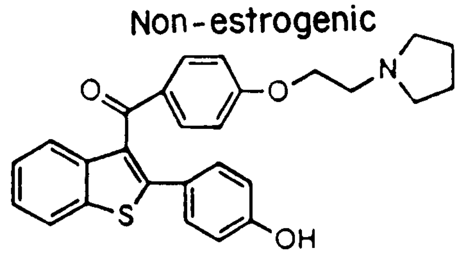

113526

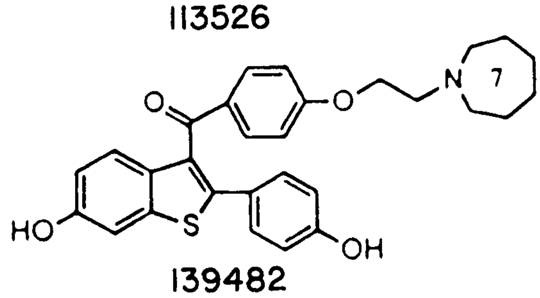

139482

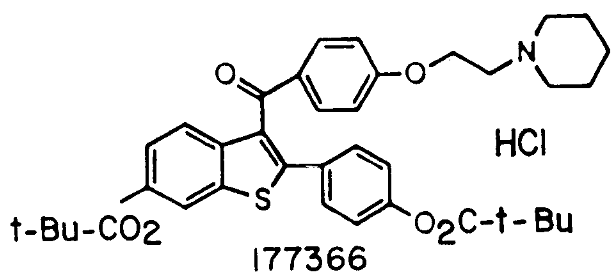

177366

Inactive

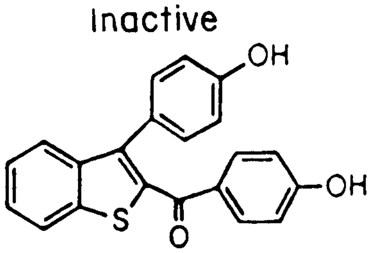

98005

Raloxifene

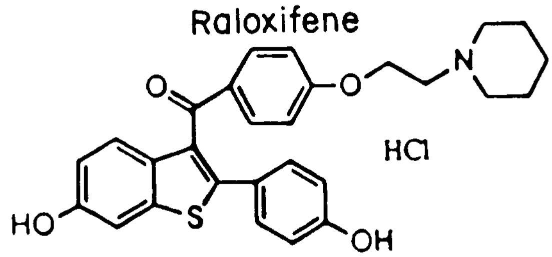

156758

FIG. 12

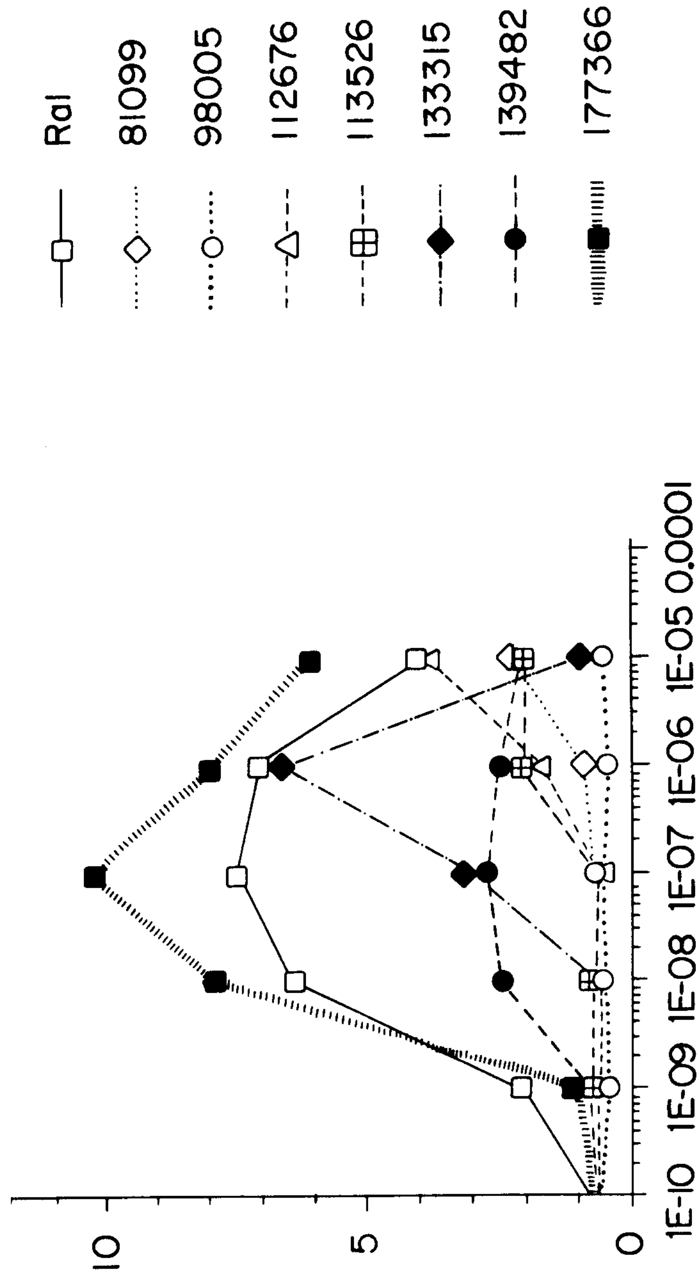
CAT Activity
10
5
0
1E-10 1E-09 1E-08 1E-07 1E-06 1E-05 0.0001
CONCENTRATION (M)
Ral
81099
98005
112676
113526
133315
139482
177366

FIG. 13

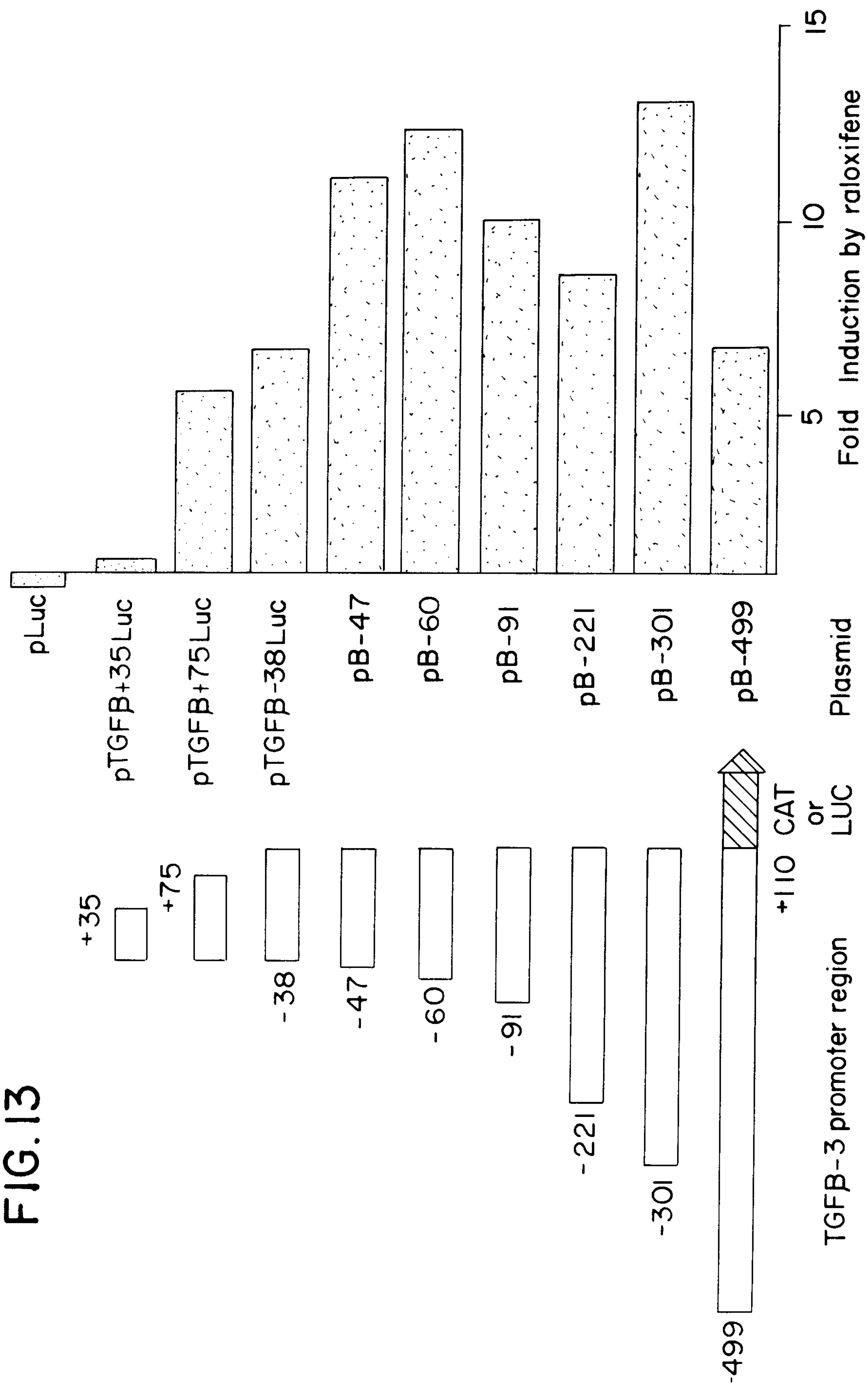

+35
+75
-38
-47
-60
-91
-221
-301
-499
+110
CAT
or
LUC
TGFβ-3 promoter region
pLuc
pTGFβ+35Luc
pTGFβ+75Luc
pTGFβ-38Luc
pB-47
pB-60
pB-91
pB-221
pB-301
pB-499
Plasmid
5
10
15
Fold Induction by raloxifene

# FIG.14

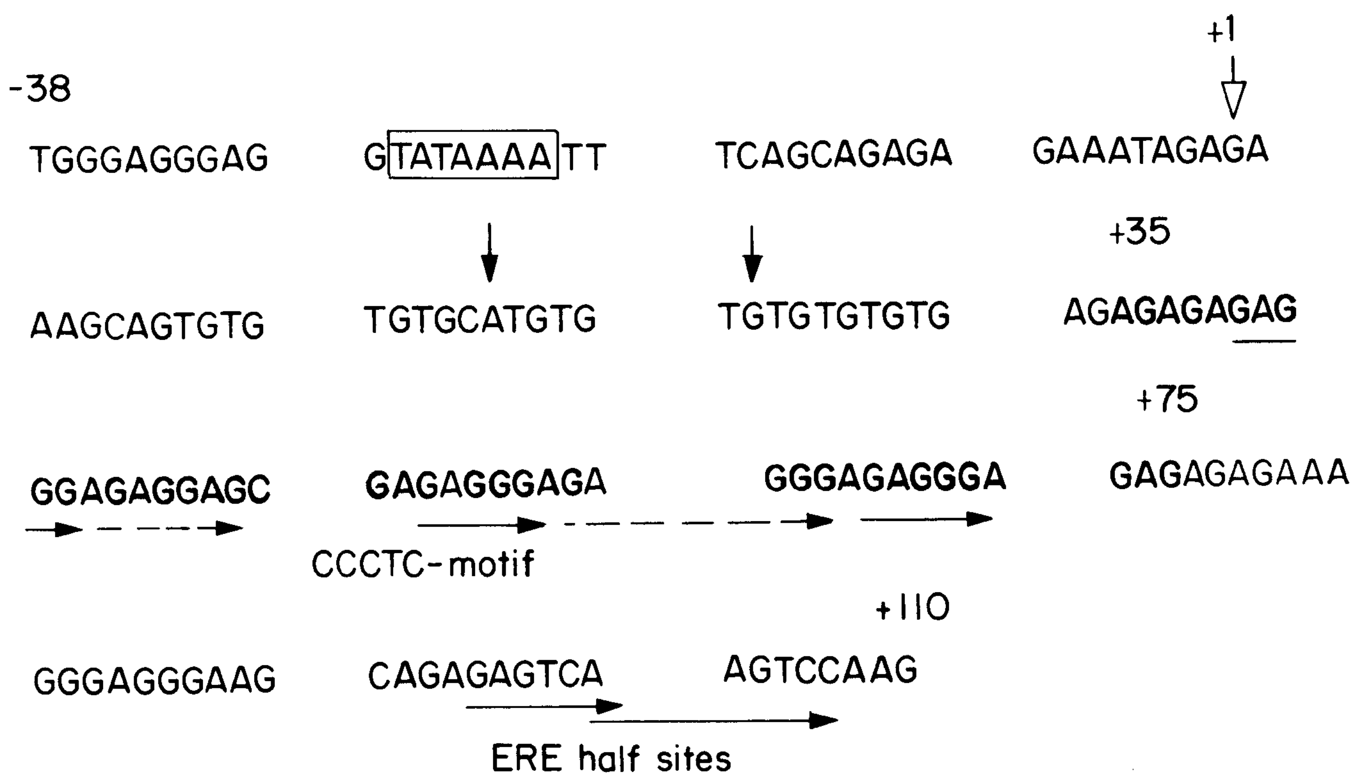
+1
-38
TGGGAGGGAG
GTATAAAATT
TCAGCAGAGA
GAAATAGAGA
+35
AAGCAGTGTG
TGTGCATGTG
TGTGTGTGTG
AGAGAGAGAG
+75
GGAGAGGAGC
GAGAGGGAGA
GGGAGAGGGA
GAGAGAGAAA
CCCTC-motif
+110
GGGAGGGAAG
CAGAGAGTCA
AGTCCAAG
ERE half sites

FIG.15

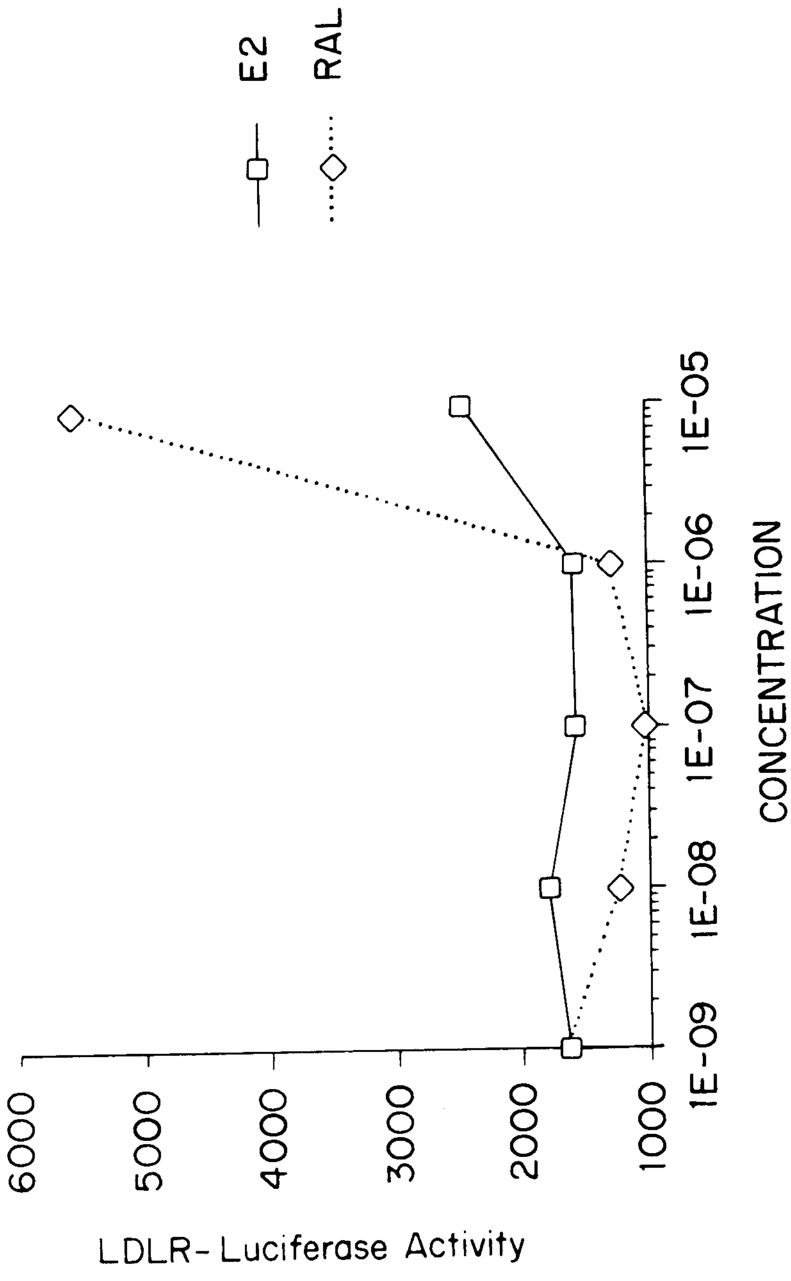
E2
RAL
6000
5000
4000
3000
2000
1000
LDLR- Luciferase Activity
1E-09
1E-08
1E-07
1E-06
1E-05
CONCENTRATION

FIG.16

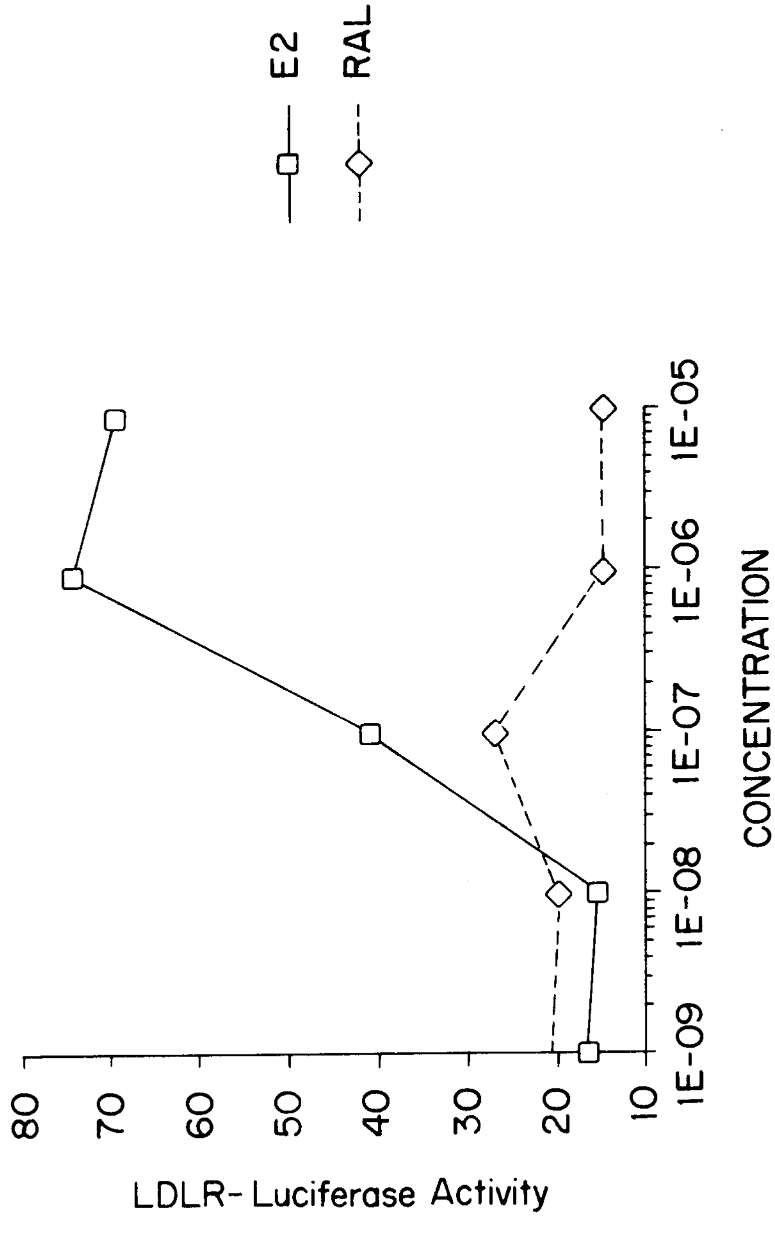
E2
RAL
80
70
60
50
40
30
20
10
LDLR- Luciferase Activity
1E-09
1E-08
1E-07
1E-06
1E-05
CONCENTRATION

FIG. 17

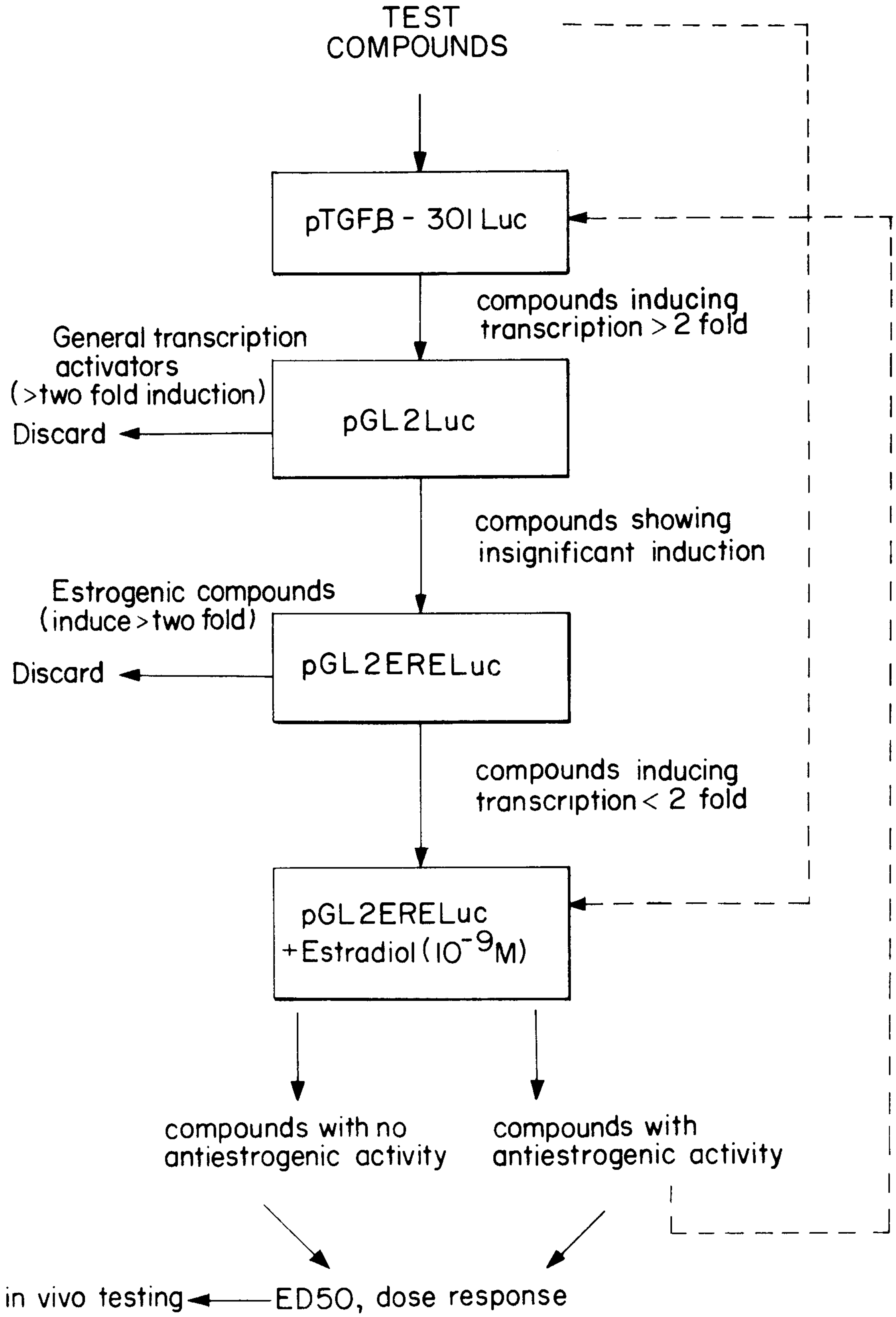
TEST
COMPOUNDS
pTGFβ - 301 Luc
compounds inducing
transcription > 2 fold
General transcription
activators
(>two fold induction)
Discard
pGL2Luc
compounds showing
insignificant induction
Estrogenic compounds
(induce > two fold)
Discard
pGL2ERELuc
compounds inducing
transcription < 2 fold
pGL2ERELuc
+Estradiol ($10^{-9}$M)
compounds with no
antiestrogenic activity
compounds with
antiestrogenic activity
in vivo testing
ED50, dose response

SEQUENCE LISTING

(1) GENERAL INFORMATION:

(i) APPLICANT: ELI LILLY AND COMPANY
(B) STREET: Lilly Corporate Center
(C) CITY: Indianapolis
(D) STATE: Indiana
(E) COUNTRY: United States of America
(F) ZIP: 46285

(ii) TITLE OF INVENTION: MATERIALS AND METHODS FOR SCREENING ANTI-OSTEOPOROSIS

(iii) NUMBER OF SEQUENCES: 9

(iv) CORRESPONDENCE ADDRESS:
(A) ADDRESSEE: C. M. Hudson
(B) STREET: Erl Wood Manor
(C) CITY: Windlesham
(D) STATE: Surrey
(E) COUNTRY: United Kingdom
(F) ZIP: GU20 6PH

(v) COMPUTER READABLE FORM:
(A) MEDIUM TYPE: Floppy disk
(B) COMPUTER: Macintosh
(C) OPERATING SYSTEM: Macintosh 7.0
(D) SOFTWARE: Microsoft Word 5.1

(2) INFORMATION FOR SEQ ID NO:1:

(i) SEQUENCE CHARACTERISTICS:
(A) LENGTH: 2205 base pairs
(B) TYPE: nucleic acid
(C) STRANDEDNESS: single
(D) TOPOLOGY: linear

(ii) MOLECULAR TYPE: cDNA

(ix) FEATURE:
(A) NAME/KEY: misc_RNA
(B) LOCATION: 1..2
(D) OTHER INFORMATION: /note= "Number 1 corresponds to -1362 of TGFB-1 promoter"

(ix) FEATURE:
(A) NAME/KEY: misc_RNA
(B) LOCATION: 1363..1365
(D) OTHER INFORMATION: /note= "Corresponds to +1 codon of TGFB-1"

(xi) SEQUENCE DESCRIPTION: SEQ ID NO:1:

GGATCCTTAG CAGGGGAGTA ACATGGATTT GGAAAGATCA CTTTGGCTGC TGTGTGGGGA 60

TAGATAAGAC GGTGGGAGCC TAGAAAGGAG GCTGGGTTGG AAACTCTGGG ACAGAAACCC 120

AGAGAGGAAA AGACTGGGCC TGGGGTCTCC AGTGAGTATC AGGGAGTGGG GAATCAGCAG 180

GAGTCTGGTC CCCACCCATC CCTCCTTTCC CCTCTCTCTC CTTTCCTGCA GGCTGGCCCC 240

GGCTCCATTT CCAGGTGTGG TCCCAGGACA GCTTTGGCCG CTGCCAGCTT GCAGGCTATG 300

GATTTTGCCA TGTGCCCAGT AGCCCGGGCA CCCACCAGCT GGCCTGCCCC ACGTGGCGGC 360

CCCTGGGCAG TTGGCGAGAA CAGTTGGCAC GGGCTTTCGT GGGTGGTGGG CCGCAGCTGC 420

TGCATGGGGA CACCATCTAC AGTGGGGCCG ACCGCTATCG CCTGCACACA GCTGCTGGTG 480

GCACCGTGCA CCTGGAGATC GGCCTGCTGC TCCGCAACTT CGACCGCTAC GGCGTGGAGT 540

GCTGAGGGAC TCTGCCTCCA ACGTCACCAC CATCCACACC CCGGACACCC AGTGATGGGG 600

GAGGATGGCA CAGTGGTCAA GAGCACAGAC TCTAGAGACT GTCAGAGCTG ACCCCAGCTA 660

AGGCATGGCA CCGCTTCTGT CCTTTCTAGG ACCTCGGGGT CCCTCTGGGC CCAGTTTCCC 720

TATCTGTAAA TTGGGGACAG TAAATGTATG GGGTCGCAGG GTGTTGAGTG ACAGGAGGCT 780

GCTTAGCCAC ATGGGAGGTG CTCAGTAAAG GAGAGCAATT CTTACAGGTG TCTGCCTCCT 840

GACCCTTCCA TCCCTCAGGT GTCCTGTTGC CCCCTCCTCC CACTGACACC CTCCGGAGGC 900

CCCCATGTTG ACAGACCCTC CTTCTCCTAC CTTGTTTCCC AGCCTGACTC TCCTTCCGTT 960

CTGGGTCCCC CTCCTCTGGT CGGCTCCCCT GTGTCTCATC CCCCGGATTA AGCCTTCTCC 1020

GCCTGGTCCT CTTTCTCTGG TGACCCACAC CGCCCGCAAA GCCACAGCGC ATCTGGATCA 1080

CCCGCTTTGG TGGCGCTTGG CCGCCAGGAG GCAGCACCCT GTTTGCGGGG CGGAGCCGGG 1140

GAGCCCGCCC CCTTTCCCCC AGGGCTGAAG GGACCCCCCT CGGAGCCCGC CCACGCGAGA 1200

TGAGGACGGT GGCCCAGCCC CCCCATGCCC TCCCCCTGGG GGCCGCCCCC GCTCCCGCCC 1260

```
CGTGCGCTTC CTGGGTGGGG CCGGGGGCGG CTTCAAAACC CCCTGCCGAC CCAGCCGGTC    1320
CCCGCCGCCG CCGCCCTTCG CGCCCTGGGC CATCTCCCTC CCACCTCCCT CCGCGGAGCA    1380
GCCAGACAGC GAGGGCCCCG GCCGGGGGCA GGGGGGACGC CCCGTCCGGG GCACCCCCCC    1440
GGCTCTGAGC CGCCCGCGGG GCCGGCCTCG GCCCGGAGCG GAGGAAGGAG TCGCCGAGGA    1500
GCAGCCTGAG GCCCCAGAGT CTGAGACGAG CCGCCGCCGC CCCCGCCACT GCGGGGAGGA    1560
GGGGGAGGAG GAGCGGGAGG AGGGACGAGC TGGTCGGGAG AAGAGGAAAA AAACTTTTGA    1620
GACTTTTCCG TTGCCGCTGG GAGCCGGAGG CGCGGGGACC TCTTGGCGCG ACGCTGCCCC    1680
GCGAGGAGGC AGGACTTGGG GACCCCAGAC CGCCTCCCTT TGCCGCCGGG GACGCTTGCT    1740
CCCTCCCTGC CCCCTACACG GCGTCCCTCA GGCGCCCCCA TTCCGGACCA GCCCTCGGGA    1800
GTCGCCGACC CGGCCTCCCG CAAAGACTTT TCCCCAGACC TCGGGCGCAC CCCCTGCACG    1860
CCGCCTTCAT CCCCGGCCTG TCTCCTGAGC CCCCGCGCAT CCTAGACCCT TTCTCCTCCA    1920
GGAGACGGAT CTCTCTCCGA CCTGCCACAG ATCCCCTATT CAAGACCACC CACCTTCTGG    1980
TACCAGATCG CGCCCATCTA GGTTATTTCC GTGGGATACT GAGACACCCC CGGTCCAAGC    2040
CTCCCCTCCA CCACTGCGCC CTTCTCCCTG AGGAGCCTCA GCTTTCCCTC GAGGCCCTCC    2100
TACCTTTTGC CGGGAGACCC CCAGCCCCTG CAGGGGCGGG GCCTCCCCAC CACACCAGCC    2160
CTGTTCGCGC TCTCGGCAGT GCCGGGGGGC GCCGCCTCCC CCATG                    2205
```

(2) INFORMATION FOR SEQ ID NO:2:

(i) SEQUENCE CHARACTERISTICS:
(A) LENGTH: 5578 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: TATA_signal
(B) LOCATION: 2248..2252

(ix) FEATURE:
(A) NAME/KEY: exon
(B) LOCATION: 2278..3980

(ix) FEATURE:
(A) NAME/KEY: intron
(B) LOCATION: 3981..5578

(ix) FEATURE:
(A) NAME/KEY: misc_RNA
(B) LOCATION: 3635..3980
(D) OTHER INFORMATION: /note= "CDS, Codon start = 1"

(ix) FEATURE:
(A) NAME/KEY: misc_RNA
(B) LOCATION: 1..2
(D) OTHER INFORMATION: /note= "Number 1 corresponds to TGFB-2 -2277"

(xi) SEQUENCE DESCRIPTION: SEQ ID NO:2:

```
AAGCTTTTAC CAATACCTCC CGTCTTACCC CTCCTGGGCT TTGGGGAAAT TAAAGTAGCC     60
TCTTATGAGT AAGTCAGGGG TCTCAGGTCT AAGGAGTTGT TAAGTGAGCA ATAGGTACTC    120
AGTAAACTAA GTATTATGAA CAAAAGTGTG TATGTCTATG TCAGGAAGAG GGGTGGCCAT    180
CAGAATTTAT GGCTTGCGCT GTTTCCTAGA AGTGATGTAA TGAACTTTTG CTACTCTATC    240
CACACTCTAA TCTGAATCTA CTTAAGGTGC ATCAGTGTCT GTACCAAGAA GGTTGTCTAT    300
AAACATGAAA GATGGATGCT CACTGGCTTG TGGAAGCTGA ACCTGTATCC TCAGAAAATA    360
CAGTGATAGC TAATTCAGGT AACCAGCCAT ATTCCACAGC AGCATCTTCT CTCAGTAGCT    420
CTGGTTTGGA GCTCCTGCTC TGTGTCTATA ATGGCCACAG GTGTAAGAAT ATTCACTTTT    480
TGTCCAATCT GTAGAGCTAG CCTACTGCAG TTCTCAAACT GAACTCAGAG GGAGGACCTA    540
ACTGGATGAA ACTACTAGTC TGACAGTAGC GCCTCTTGAT TATCTTTTTC TTGGGCTACT    600
GGGATGGTAG CTTTGCTTCA ACTCAAAACT GGTATCAAGG AAAGGAACCT GCTGGTGCTG    660
ATTTATACAT AATTTTTAGA ATTATTCAGA AGTGGGTTGG AACAATTATT TTATTCCAGA    720
GTTTTTCAAT GTGTGATAAT GGAAAAAATT CTGTATTCAA GGGAGTTTGG AAAATGCTGG    780
GTTAAAAGAG TGAAAAGGTT TTCTCTTCTA CAGGAGTTTC AGAGCCTTTA ACATGATAAT    840
GTTCCAGAAT GAGGAATCTA AGAGGACAGG AGAGTACCCA GTATCTCCCA AACTTGTTTG    900
ACTCCAGAAT TCCTGTTTGT CAGAACATAT TCTGGGACCA TTGTTTCTCA GAAGTACATA    960
GTAGGTAAGA ACATAGTGGA TCCTGACTGC AAAAATCCAG CTCTACCACT TACTGTGGTC   1020
TCGAACAAAG TACTTAACTT CTTTGTACCT CAGTCTCCTC ATCTGCCAGA TATGGATAAT   1080
AAGACCCACT TTATAGGTTC ATAGTGAAGA TTAAATGACC ATACACAACA CACATCAAAT   1140
TACTAAGTGT AGTATATGTT AGCTATTATT ATTTTATTTA TTCAGTGCTC TACTAATAAC   1200
CTAGGCCCCA TACACAACTG AAGTATAATT CCAAAAGTGA TAGAAAGTTC TTTGTGACTT   1260
TTCTGAACTC AGGAACATCT GAAGTAGAGA ACAGTATAGA GATCTTGGGT TTGGGAGTAC   1320
```

ATTCAACAGA GTTTTCCAGT TTAAATCATC TGTCTGGTCA GTATGGCTGC AGAGTCATGC 1380

CGAAATGAAA ATGTTGACTT TGAGTAACTA AAGGTAAAAT AAAAGAAAAA GGGAAGGTGG 1440

AACAGTGGTA AGAGTTATTC TGTATTCATC TAATTTAAGA CTTAGTTGAA ATTGAAAATG 1500

TCAAGTTATG AGTAGTGTAG AACAAGTAGA CATCAAACAC TTAAAATTCC AGCTTCCTGG 1560

ATTATGCTAT GGAAAGAATG AAGTTGGTGG ATAATGTTTA GCCTAGCAAG AAGGTCAAGA 1620

AGAAGAAAGC CATACAAGAA GTGGCTTAGG CAGCAAATTA TAAAGGTGAC CATTCATTCA 1680

AATCAGTAAA ACAAACAAGT ATACCTTATT CTTTAGGTAA AATTGATGGA TCTCTGTTTT 1740

CCAGCAGTTC ACAAACAGAG GGGTACATTG TAAACAACAA ACTAACAAAA TAAATTCTGG 1800

GATGGCAACC TGCTAAGGTA TCCCAGAAAA TAAGAGGTAG GACATGAATT TAAAAGATTG 1860

GAAGGTATGT CTTCAGTACT GGCCTGGCCC TGAGTAGACT AGTGCCCTCC ATAGGGGTGC 1920

GTGTGCACAC ATAATACAGG AGGGAAGCCT TCCCTTCTAG AGCAAGTGAT TCAGCTTGGG 1980

AGGCTGTGAC TGAGCTACAC TAAGTAAAAA CGGGAGACTT GATTGTCCTT CAACAGACCT 2040

GTCCAAAATG ACTGGAAAGT AAATACCGTA AATCACTGTT GTCAGGGCGC ACATTCCACC 2100

TCCTTCCTCC CTTACCCACA GCGGTCCACA TTTCCACACT CCCTACACGG TTCGGGGAGA 2160

GCTCGTGGTC TAAGTAACGA GAGGACTTCT GACTGTAATC CTAGCACGTC ACTTTGTTGA 2220

AGGCAGACAC GTGGTTCAGA GAGAACTTAT AAATCTCCCC TCCCCGCGAA GATCGTGATG 2280

TTATTCGCTG GCAGCAGAAG GTCTTGCCGA GCGAGCTCCA GAACGTCCTG ACAAGAGAAA 2340

GACAGATTGA GATAGAGATA GAAAGAGAAA GAGAGAAAGA GACAGCAGAG CGAGAGCGCA 2400

AGTGAAAGAG GCAGGGGAGG AGGGGGATGG AGCATATTAC GTGACCGGCC TAGGGAGTCA 2460

TCCAGGAACA AACTGAGGGG CTGCCCGGCT GCAGACAGGA GGAGACAGAG AGGATCTATT 2520

TTAGGGTGGC AAGTGCCTCC TACCCTAAGC GAGCAATTCC ACGTTGGGGA GAAGCCAGCA 2580

GAGGTTGGGA AAGGGTGGGA GTCCAAGGGA CGCCCCTGCG CAACTCCCTC AGGAATAAAA 2640

CTCCCCAGCC AGGGTGTCGC AAGGGCTGCC GTTGTGATCC GCAGGGGGTG AACGCAACCG 2700

CGACGGCTGA TCGTATGTGG CTGGGTTGGC GTTTGGAGCA AGAGAAGGAG GAGCAGGAGA 2760

AGGAGGGAGC TGGAGGCTGG AAGCGTTTGC AAGCGGCGGC GGCAGCAACG TGGAGTAACC 2820

AAGCGGGTCA GCGCGCGCGC GCCAGGGTGT AGGCCACGGC GCGCAGCTCC CAGAGCAGGA 2880

TCCGCCCGCC CTCGGCAGCC TCTGCGGCCC CTGCGGCACC CGACCGAGTA CCGAGCGCCC 2940

TGCGAACGGC ACCCTCCTCC CCGCGGTGGC TGGGCTCGCC CCAGCGCGCA CACGCACACA 3000

CACACACACA CACACACACG CACGCACACA CGTGTCGTTC TCTGCTCCGG AGCTGCTGCT 3060
GCTCCTGCTC TCAGCGCCGC AGTGGAAGGC AGGACCGAAC CGCTCCTTCT TTAAATATAT 3120
AAATTTCAGC CCAGGTCAGC CTCGGCGGCC CCCCTCACCG CGCTCCCGCC CCTCCCGTCA 3180
GTTCGCCAGC TGCCAGCCCC GGGACCTTTT CATCTCTTCC CTTTTGGCCG GAGGAGCCGA 3240
GTTCAGATCC GCCACTCCGC ACCCGAGACT GACACACTGA ACTCCACTTC CTCCTCTTAA 3300
ATTTATTTCT ACTTAATAGC CACTCGTCTC TTTTTTTCCC CATCTCATTG CTCCAAGAAT 3360
TTTTTTCTTC TTACTCGCCA AAGTCAGGGT TCCCTCTGCC CGTCCCCTAT TAATATTTCC 3420
ACTTTTGGAA CTACTGGCCT TTTCTTTTTA AAGGAATTCA AGCAGGATAC GTTTTTCTGT 3480
TGGGCATTGA CTAGATTGTT TGCAAAAGTT TCGCATCAAA AACAACAACA ACAAAAAACC 3540
AAACAACTCT CCTTGATCTA TACTTTGAGA ATTGTTGATT TCTTTTTTTT ATTCTGACTT 3600
TTAAAAACAA CTTTTTTTTC CACTTTTTTA AAAAATGCAC TACTGTGTGC TGAGCGCTTT 3660
TCTGATCCTG CATCTGGTCA CGGTCGCGCT CAGCCTGTCT ACCTGCAGCA CACTCGATAT 3720
GGACCAGCTC ATGCGCAAGA GGATCGAGGC GATCCGCGGG CAGATCCTGA GCAAGCTGAA 3780
GCTCACCAGT CCCCCAGAAG ACTATCCTGA GCCCGAGGAA GTCCCCCCGG AGGTGATTTC 3840
CATCTACAAC AGCACCAGGG ACTTGCTCCA GGAGAAGGCG AGCCGGAGGG CGGCCGCCTG 3900
CGAGCGCGAG AGGAGCGACG AAGAGTACTA CGCCAAGGAG GTTTACAAAA TAGACATGCC 3960
GCCCTTCTTC CCCTCCGAAA GTAAGTACTT ATTTTGACTT CCATCCCCTG AGGTTTAGCT 4020
CTGCCCGGAG CTCTCAAAAC CGCAGCAGCT CCCGGGATCG CCCTTCCCTC TGCCGGTTCC 4080
CGTTCGCTCT TTTCCCGTTC TCCTGTCCTT CACCCCACCA CCTCCTTTTC AGTTGTAGTC 4140
TTGAGGCCAT CAGGCTTTAA AATGTTTACT TTCTACTTTA TTTTCTCCAT CTTTCCCTTG 4200
CCCCCTAACA ATGCGGTTCT TTAAAAGGCG TTATTCTCTT TTTCTCTTCC CTGAAGTTCT 4260
TTAGTCGGCC ACCAGCTAAG GAGTCAGCCC CACTCTGTCA AACTAGAGGT GCTCCCAGGG 4320
GCAGAGTTAA ACTGAGGAAT CTTCGTAGGT TTGTTTTCTT TGCTCCGATT GGCGTGGAGC 4380
GGCCGAACTG GTGCACGAGG GTTAAAAAAA GTGCTCTCAA AACTAGCCTC TGCCGGAAGC 4440
GCCCCCTTTC CGTGCTGACC TATCAGCTGG TTCCCCAAGC CTTCTCTATT GTCTCTAACT 4500
ACCCTAAAAA TGTCAGCATC GCCGAGACAA AACCCGGTTT GGAGACCCCT CGAGAAACCT 4560
ACCTGGCCCT CAGTCCTTGA TGTATACTTT GCTATACCTA GGTGTTTCAT TACCCACCGG 4620
CAAAATCCTA TAACCACGTT CCCTTTTCAC TTAACCTGGA GCGCAGAAAG GACAACTCCG 4680

```
TTTCTGACTA TGTTTTAAAA GGTTTTGTTC ACGTTATTTT TCAGCATACA CTCAAACCTG    4740
CCTTCTTCAC ATCTCCAGTG TAGCAGATCA TTTTTCTTAC GGGTCTGTTA TCCTGCTCCT    4800
GCCTTTTCGT AGGCTTCCTG CAGTTACTTC AATGCATTCT TAAAACTCAG AGTAGACGAC    4860
AGCCGTATTT TTTTTTTTTT TTTTTACTGG CTTCTCTGAG AACAGTGTCC TCAAAACCAG    4920
CTGGCATACA GTAGCAATAG GAGTGAAATG ATTTATTGCA GAGGAAGGGA ACAGACAGTG    4980
TAGAATGATT TCAGAGTTCT TAAAAAAAGA AAAAAAAGAA AGAAAGAAAG AAAAGGGGCA    5040
GCAGCATCCA CTTGATACCT GAGAGGGTTA AATACCAGGA AGAAGAAAAA GAAAAGTGGG    5100
GGCGGGGTGG GGGGAACTCT TCAACATTTG TGTATTCCAA ATCCAAGTCA TAAACTTTTC    5160
ATTGGTTGCT CATTTCTCTC CTCCCCTTTC CATGCCCTAT ATACTTGCTG GCTGCCTTTG    5220
CAAAGTCTCT GTGTCTTGCC TAAATAGATA ATATAGCCTT CTTGGTAATT TTCTCTTAAA    5280
GGTTCTAGTT GCAGGGTGGT GCTTTTCTTT TTTAATATTT ATTTTTAGTT TGACAAGTCC    5340
TAGCTATGTG ACCTGCCATG TCTTGTACTT GATGGTCTCA GAAGTCAGCC CATGTATCTA    5400
ACCCCAGTCT TCCTAGTGAC CCTTATTTTG CTGCAGTTTC TCCTGTTCTT GTTCAATAGC    5460
AGAACAGATG CAGAGAATTC TGGCAAGCAG GATGATTTTA TTATTGTAAT TATGGCACTA    5520
TCCGCAACAG CTGATAAATA CACTCCACCC CTGGTTATCC CCTTTGGAAG TAAAGCTT      5578
```

(2) INFORMATION FOR SEQ ID NO:3:

(i) SEQUENCE CHARACTERISTICS:
(A) LENGTH: 3303 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: mRNA
(B) LOCATION: 2170..3303

(ix) FEATURE:
(A) NAME/KEY: mRNA
(B) LOCATION: 2214..3303

(ix) FEATURE:
(A) NAME/KEY: mRNA
(B) LOCATION: 2219..3303

(ix) FEATURE:
(A) NAME/KEY: misc_RNA
(B) LOCATION: 3301..3303
(D) OTHER INFORMATION: /note= "CDS Start, codon start = 1, translation M"

(ix) FEATURE:
(A) NAME/KEY: TATA_signal
(B) LOCATION: 2170..2176

(ix) FEATURE:
(A) NAME/KEY: misc_feature
(B) LOCATION: 1896..2306
(D) OTHER INFORMATION: /note= "pB-301   -301 to +110"

(ix) FEATURE:
(A) NAME/KEY: misc_feature
(B) LOCATION: 1976..2306
(D) OTHER INFORMATION: /note= "pB-221   -221 to +110"

(ix) FEATURE:
(A) NAME/KEY: misc_feature
(B) LOCATION: 2106..2306
(D) OTHER INFORMATION: /note= "pB-91  -91 to +110"

(ix) FEATURE:
(A) NAME/KEY: misc_feature
(B) LOCATION: 2137..2306
(D) OTHER INFORMATION: /note= "pB-60   -60 to +110"

(ix) FEATURE:
(A) NAME/KEY: misc_feature
(B) LOCATION: 2150..2306
(D) OTHER INFORMATION: /note= "pB-47   -47 to +110"

(ix) FEATURE:
(A) NAME/KEY: misc_feature
(B) LOCATION: 2159..2306
(D) OTHER INFORMATION: /note= "pB-38   -38 to +110"

(ix) FEATURE:
(A) NAME/KEY: misc_feature
(B) LOCATION: 2159..2271
(D) OTHER INFORMATION: /note= "TGFB-3 position  -38 to +75"

(ix) FEATURE:
(A) NAME/KEY: misc_feature
(B) LOCATION: 2159..2231
(D) OTHER INFORMATION: /note= "TGFB-3 position  -38 to +35"

(xi) SEQUENCE DESCRIPTION: SEQ ID NO:3:

```
CAGTAGTAGC TTCCAGAACT TGCTTAGCAC CTGAATCACG TGTGAGGTTT GTAAAGAAAC     60
AGAGATGCCA GGGCCTCAGC TCTGGAGACT GATTGGTAGA GGTGGAGTCC AAAAAAGTAT    120
AACTTTAATA ATTTTCCTTC CTATCTTCAA CTGTCTGCTC AAAGGCCTTC CCTTATCACC    180
CTATTTGAAA CTGCAACATC CCCCAACCTA GGCACACCCC ATCCTCCTTC CCTGCTTGAT    240
TTTCTGCCAC ACCACATTTG TTTGTTTGCT TGTCTGTTTG AGACACGGTC TTGCTCTGTC    300
GTCCAGGCTG GAGTGCAGTG GTGCAATCTT GGCCCCCTGT AAACTCGCCT CCCTGGCTCA    360
AGTGATTATC CTGCTCAGCC TCCCAAGTAG ATGCGTGCGC CAACATGCCG GGCTAATTTT    420
TCCATTTTTT TGTAGAGACT GGGTTTCGCC GTGTTGCTGG GGCTGGTCTC GAATTCCTGA    480
GCTCAAGTAA TCCTCCTGCA TGGGCCTCCC CAAATGCTGG GATTACAGGC GTGAGCCACT    540
GCACCTGGCT CAGCACTTTT TACCGTACTA CATCATTTAC ATATTTATTT AGTTTATCGC    600
CTCCTCCACT GCCCCACCCC TGCCTCTAAA TAAAATTTCC CTGAGGGCAG GAGTTTTGTT    660
TCGTTCACTG ATATTCTTCA CAGAGCCTAG AATAGTGCCT GGTATATAGA AACATTAAAC    720
TTTTTCTGAA ATTTCAGAGG CAGTATAGCA TAGTAATTAA GTCCAGAATC TGGCAACGTC    780
CTGGGTGCAA ATCCCAACAG CTGACACCTA ATAACTATGT GACCTTGGGC AAGTTACTTT    840
TAAAGTTTCT ACCCCTAGGT TTCCCATTGG TTTTGCAAAT GAAAGTAATG CCTACCCAAG    900
CTAGATAGCC TGTGTAAATA TCGCCTCCAT CACTCACAAG CAGTGTGGTC TGTAAAAAAA    960
AAAACAAAAA ACTCTATGCC TCAGTTTCCT CATCCGTAAA AGTGACCCAC CGCTGTGCTG   1020
GGATACAGAG AACAGCCCCT TCAGTTAGTG GCCTGGAAGC CAGCCTCTCA GAAAGGGTCC   1080
AGGAAGGCTG GAGTGAGATG GGGTGGAGCG GCACTCACTC TCAGGAAAGT TCAGTTCAGA   1140
GGCAAGCCCT GTGTTGCGGG GTGCGGGGAG CCACGTGCCC TACCCTCCCT TGGCTGCTCG   1200
TGGGAAAAGG CCTAGAGGTT CGGGCCGAGA AGAGGAGCGA AAGCACAGAG CCGACTTCCC   1260
CTCACCCATC TGGGAAATGG CTCGGGCCAA CTGCTGACTT CGCGCTCGCT GGCCGACGTC   1320
CTGCGGAGAC CTCGGCGGGG AGGGAGGCTG AACATCTGGA TGACATTTCT GCGAGAGAGC   1380
GGCTCCGGAG CGGCGGTCGG GGAGGGAGAG CTGCTCGTGC GCACGTCGGG CCGGGAGGGA   1440
GGCGATTCCT CGGGGCCTGG GTCTTGTTTT TCTCGCTCTC TACCGCAGCC CCTTCTCCCG   1500
CCCCTCAGCC CCCACCCCGC AGCCCCCAGC CCCCGAGCCT CCCCGGCTCC CGACCAGCCG   1560
AGCTCCTTCA CTGGCGGCCT CCGCTCGCCA GAGGGCACCC TCGATCTTCC GGAAAACGCC   1620
```

ACCATTTTTC ACTGCCCCTG GAGCGTCTCC AGGCTTCTGC CCGCCTCCCG ACTCCGATCT 1680
TGTCAATGAA GAATCGGGCC AGGATCGCCG CGGAGCGGAC GCCGACCCTC CGACCCGGCT 1740
CGCAGGCTGG GAGTCCCCTC TGCGAGGCTG GCATGGCCGC CCCTACCGGG TCCCGCGCCC 1800
TCTGCGGACC CTCCCCGGGT TGGGCCTGGC CGCGGGCGGC CCCGGGACCG GGGGACCAGG 1860
AGGGAGAGTA GACCGGGCCG GACGGCGCGG ACTGACAGCT GGCGAGAGGG CGCCGGGGCT 1920
GGGGGAAAGG GAGGGAGGGG GCTCATCGGA GTAACTTTCC AGAAAAACAC CAACGTGTGG 1980
CAGGAGTGAT TCCAAGAGGG GAAAAAAAGT TCAGCTACCA CGTCGAACGA GAGGACTCGC 2040
AAAGTATTTT TCAAAAGGGC TCGGCTTTTC CTGTGCCTGT TTAAAACATT AACATCGTGC 2100
AGCAAAAGAG GCTGCGTGCG CTGGTCCCTC CCTCCCCCAC CCCAGGCCAG AGACGTCATG 2160
GGAGGGAGGT ATAAAATTTC AGCAGAGAGA AATAGAGAAA GCAGTGTGTG TGCATGTGTG 2220
TGTGTGTGAG AGAGAGAGGG AGAGGAGCGA GAGGGAGAGG GAGAGGGAGA GAGAGAAAGG 2280
GAGGGAAGCA GAGAGTCAAG TCCAAGGGAA TGACCGAGAG AGGCAGAGAC AGGGGAAGAG 2340
GCGTGCGAGA GAAGGAATAA CAGCAGCTTT CCGGAGCAGG CGTGCCGTGA ACTGGCTTCT 2400
ATTTTATTTT ATTTTTTTCT CCTTTTTATT TTTTAAAGAG AAGCAGGGGA CAGAAGCAAT 2460
GGCCGAGGCA GAAGACAAGC CGAGGTGCTG GTGACCCTGG GCGTCTGAGT GGATGATTGG 2520
GGCTGCTGCG CTCAGAGGCC TGCCTCCCTG CCTTCCAATG CATATAACCC CACACCCCAG 2580
CCAATGAAGA CGAGAGGCAG CTGAAAAAGT CATTTAGAAA GCCCCCGAGG AAGTGTAAAC 2640
AAAAGAGAAA GCATGAATGG AGTGCCTGAG AGACAAGTGT GTCCTGTACT GCCCCACCTT 2700
TAGCTGGGCC AGCAACTGCC CGGCCCGCTT CTCCCCACCT ACTCACTGGT GATCTTTTTT 2760
TTTTTACTTT TTTTTCCCTT TTCTTTTCCA TTCTCTTTTC TTATTTTCTT TCAAGGCAAG 2820
GCAAGGATTT TGATTTTGGG ACCCAGCCAT GGTCCTTCTG CTTCTTCTTT AAAATACCCA 2880
CTTTCTCCCC ATCGCCAAGC GGCGTTTGGC AATATCAGAT ATCCACTCTA TTTATTTTTA 2940
CCTAAGGAAA AACTCCAGCT CCCTTCCCAC TCCCAGCTGC CTTGCCACCC CTCCCAGCCC 3000
TCTGCTTGCC CTCCACCTGG CCTGCTGGGA GTCAGAGCCC AGCAAAACCT GTTTAGACAC 3060
ATGGACAAGA ATCCCAGCGC TACAAGGCAC ACAGTCCGCT TCTTCGTCCT CAGGGTTGCC 3120
AGCGCTTCCT GGAAGTCCTG AAGCTCTCGC AGTGCAGTGA GTTCATGCAC CTTCTTGCCA 3180

AGCCTCAGTC TTTGGGATCT GGGGAGGCCG CCTGGTTTTC CTCCCTCCTT CTGCACGTCT 3240

GCTGGGGTCT CTTCCTCTCC AGGCCTTGCC GTCCCCCTGG CCTCTCTTCC CAGCTCACAC 3300

ATG 3303

(2) INFORMATION FOR SEQ ID NO:4:

(i) SEQUENCE CHARACTERISTICS:
(A) LENGTH: 42 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:4:

GCGCCATATG AGTCTTAACT GCCAAAAATT CTTATCATCA AT 42

(2) INFORMATION FOR SEQ ID NO:5:

(i) SEQUENCE CHARACTERISTICS:
(A) LENGTH: 44 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:5:

AAGCAAGCTT TCGCAGCCTC TGCCAGGCAG TGTCCCGACC CGGA 44

(2) INFORMATION FOR SEQ ID NO:6:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:6:

AGCTTCGCGA CTCGAGA 17

(2) INFORMATION FOR SEQ ID NO:7:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:7:

GATCTCTCCA GTCGCGA 17

(2) INFORMATION FOR SEQ ID NO:8:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:8:

TCGAGAAAAG TCAGGTCACA GTGACCTGAT CAAAC 35

(2) INFORMATION FOR SEQ ID NO:9:

(i) SEQUENCE CHARACTERISTICS:
(A) LENGTH: 35 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:9:

TCGAGTTTGA TCAGGTCACT GTGACCTGAC TTTTC 35